# FASCICULE DE BREVET EUROPEEN

(11) **EP 2 198 837 B1**
(45) Date de publication et mention de la délivrance du brevet: **17.02.2016**
(21) Numéro de dépôt: 09306254.5
(22) Date de dépôt: 17.12.2009
(51) Int. Cl.: A61K 8/27, A61K 8/35, A61K 8/39, A61K 8/44, A61K 8/46, A61K 8/60, A61K 8/81, A61Q 5/02, A61Q 5/00

(54) **Composition cosmétique détergente comprenant quatre tensioactifs, un polymère cationique et un sel de zinc**
Kosmetische Zusammensetzung enthaltend vier Tenside, ein kationisches Polymer und ein Zinksalz
Cosmetic composition containing four surfactants, a cationic polymer and a zinc salt

(30) Priorité: 22.12.2008 FR 0858956
(43) Date de publication de la demande: 23.06.2010
(73) Titulaire: L'Oréal, 75008 Paris (FR)
(72) Inventeur: Lalleman, Boris, 75004, Paris (FR)
(74) Mandataire: Dossmann, Gérard

(56) Documents cités:
- EP-A- 1 504 749
- EP-A- 1 541 120
- EP-A- 1 674 133
- EP-A- 1 915 981
- WO-A-99/13844
- WO-A1-96/09030
- JP-A- 2003 095 897
- US-A1- 2003 150 069

## Description

La présente invention concerne de nouvelles compositions cosmétiques détergentes destinées au lavage et/ou nettoyage des fibres kératiniques, en particulier des fibres kératiniques humaines telles que les cheveux, notamment colorées comprenant un ou plusieurs polymères cationiques et un ou plusieurs sels de zinc dans des proportions particulières. L'invention concerne aussi leurs différentes utilisations, notamment pour protéger la couleur artificielle des fibres kératiniques des lavages répétés.

Il est connu de teindre les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les cheveux par une coloration directe. Le procédé classiquement utilisé en coloration directe consiste à appliquer sur les cheveux des colorants directs qui sont des molécules colorées et colorantes ayant une affinité pour les cheveux, à laisser poser, puis à rincer les fibres.

Les colorations qui en résultent sont des colorations particulièrement chromatiques qui sont cependant temporaires ou semi-permanentes car la nature des interactions qui lient les colorants directs à la fibre kératinique et leur désorption de la surface et/ou du coeur de la fibre sont responsables de leur faible puissance tinctoriale et de leur mauvaise tenue aux lavages.

La couleur artificielle des cheveux apportée par un traitement de coloration directe ou d'oxydation s'estompe progressivement du fait des lavages répétés et de l'exposition à la lumière conduisant dans le temps à un affadissement de la coloration des cheveux.

WO 99/13844 décrit des compositions de soin capillaire comprenant un azurant optique particulier permettant de protéger les cheveux des rayons UV de la lumière et de réduire les dégradations de l'aspect du cheveu telles que les altérations et affadissements de la couleur originale.

EP 1 915 981 porte sur un procédé pour protéger durablement la couleur des fibres kératiniques teintes artificiellement vis-à-vis du lavage et des agents atmosphériques.

L'utilisation des produits ou soins rincés n'améliore pas suffisamment la tenue de la couleur artificielle des cheveux.

Il existe donc un besoin de trouver des compositions cosmétiques détergentes permettant d'améliorer la tenue de la couleur artificielle des fibres kératiniques, en particulier des fibres kératiniques humaines telles que les cheveux, colorées, face aux lavages répétés, tout en ayant des qualités d'usage satisfaisantes.

La demanderesse a découvert de manière surprenante qu'en utilisant au moins un sel de zinc dans une teneur particulière en élément zinc dans des compositions cosmétiques particulières, notamment des compositions cosmétiques détergentes, on pouvait remédier aux inconvénients cités ci-dessus, et notamment protéger de manière plus efficace et plus durable la couleur desdites fibres kératiniques contre les dégradations engendrées par la lumière, les rayonnements ultra-violet et/ou les lavages répétés (affadissement de la couleur, modification de la teinte initiale), par rapport aux produits ou soins rincés connus appliqués aux mêmes quantités, tout en obtenant de très bonnes qualités d'usage, rinçabilité, qualité du toucher après rinçage.

La présente invention a donc pour objet une composition cosmétique détergente des fibres kératiniques, en particulier des fibres kératiniques humaines telles que les cheveux, notamment colorées, comprenant, de préférence dans un milieu cosmétiquement acceptable, au moins quatre tensioactifs différents, un ou plusieurs polymères cationiques, un ou plusieurs sels de zinc organiques ou minéraux et éventuellement un ou plusieurs filtres UV dans un rapport pondéral particulier de la quantité de tensioactifs particuliers sur la quantité d'élément zinc du ou des sels de zinc.

La présente invention a aussi pour objet l'utilisation d'une composition selon l'invention dans ou pour la fabrication d'une composition cosmétique détergente des fibres kératiniques, en particulier des fibres kératiniques humaines telles que les cheveux, notamment colorées, telle que par exemple la fabrication d'un shampooing ou d'un shampooing pour cheveux colorés.

Un autre objet de l'invention concerne l'utilisation d'une composition selon l'invention pour le lavage et/ou nettoyage des fibres kératiniques, en particulier des fibres kératiniques humaines telles que les cheveux, notamment colorées.

La présente invention a également pour objet l'utilisation d'une composition selon l'invention pour protéger des lavages répétés, la couleur des fibres kératiniques, en particulier des fibres kératiniques humaines telles que les cheveux, colorées.

D'autres caractéristiques, aspects, objets et avantages de la présente invention apparaîtront encore plus clairement à la lecture de la description et des exemples qui suivent.

Les différents objets de l'invention vont maintenant être détaillés. L'ensemble des significations et définitions des composés utilisés dans la présente invention données ci-dessous sont valables pour l'ensemble des objets de l'invention.

Dans la présente demande, on qualifie une entité comme étant "anionique" lorsqu'elle possède au moins une charge négative permanente ou lorsqu'elle peut être ionisée en une entité chargée négativement, dans les conditions d'utilisation des compositions de l'invention (milieu, pH par exemple) et ne comprend pas de charge cationique.

De même, on qualifie une entité comme étant "cationique" lorsqu'elle possède au moins une charge positive permanente ou lorsqu'elle peut être ionisée en une entité chargée positivement, dans les conditions d'utilisation des compositions de l'invention (milieu, pH par exemple) et ne comprend pas de charge anionique. Cette définition s'applique aussi à un motif cationique.

On qualifie une entité comme étant "non ionique" lorsqu'elle n'est ni cationique ni anionique au sens de la présente demande, en particulier elle ne comporte aucun groupe cationique ou anionique au sens de la présente demande.

Par « cosmétiquement acceptable » ou « physiologiquement acceptable », on entend qui est compatible avec l'application sur le corps d'un être vivant, en particulier le corps humain, et notamment son cuir chevelu et ses cheveux.

Par simplification, on pourra utiliser les termes :
- "tensioactif (A)" ou "composé (A)" pour désigner un tensioactif anionique comportant dans sa structure un ou plusieurs groupes sulfate et/ou sulfonate et/ou phosphate, utilisé selon l'invention et tel que décrit ultérieurement ;
- "tensioactif (B)" ou "composé (B)" pour un tensioactif anionique carboxylique différent du tensioactif (A), utilisé selon l'invention et tel que décrit ultérieurement ;
- "tensioactif (C)" ou "composé (C)" pour un tensioactif amphotérique et/ou zwittérionique utilisé selon l'invention et tel que décrit ultérieurement ;
- "tensioactif (D)" ou "composé (D)" pour un tensioactif non ionique alkyl(poly)glycoside utilisé selon l'invention et tel que décrit ultérieurement ;
- "polymère (E)" ou "composé (E)" pour un polymère cationique utilisé selon l'invention et tel que décrit ultérieurement ;
- "composé (F)" pour un sel de zinc utilisé selon l'invention et tel que décrit ultérieurement ;
- "filtre (G)" ou "composé (G)" pour un filtre ultraviolet (noté filtre UV), utilisé selon l'invention et tel que décrit ultérieurement.

On entend par "fibres kératiniques humaines", les cheveux, les poils notamment de barbe ou moustache, les cils, les sourcils.

On entend par "fibres kératiniques colorées", des fibres kératiniques teintes artificiellement, de préférence par un procédé de coloration directe ou par un procédé de coloration d'oxydation, en présence ou non d'un agent oxydant.

On entend au sens de l'invention par "agent oxydant", tout composé ayant des propriétés oxydantes et étant différent de l'oxygène de l'air.

On entend par "lavage(s)", une ou plusieurs applications sur les fibres kératiniques d'une composition aqueuse éliminée, le plus souvent détergente telle qu'un shampooing. Cette expression inclut également les baignades, en particulier en mer ou en piscine.

Bien entendu, les composés (A), (B), (C), (D), (E), (F) et (G) tels que définis dans les différents objets de l'invention, sont chacun différents les uns des autres.

A défaut d'indication contraire, chacun des composés, optionnels ou non, utilisés ou envisagés dans le cadre de la présente invention peut être présent seul ou en mélange.

Selon un premier objet de la demande, l'invention se rapporte à une composition cosmétique détergente destinée au lavage et/ou au nettoyage des fibres kératiniques, en particulier des fibres kératiniques humaines telles que les cheveux, notamment colorées, comprenant :
- un ou plusieurs tensioactifs anioniques (A) comportant un ou plusieurs groupes sulfate et/ou sulfonate et/ou phosphate,
- un ou plusieurs tensioactifs anioniques carboxyliques (B) différents des tensioactifs anioniques cités en (A),
- un ou plusieurs tensioactifs amphotères et/ou zwittérioniques (C),
- un ou plusieurs tensioactifs non ioniques alkyl(poly)glycoside (D),
- un ou plusieurs polymères cationiques (E),
- un ou plusieurs sels de zinc (F),
- et éventuellement un ou plusieurs filtres UV (G),
et présentant un rapport pondéral de la quantité de tensioactifs anionique(s), amphotérique(s) et/ou zwittérionique(s), et non ionique(s) sur la quantité d'élément zinc dudit ou desdits sels de zinc inférieur à 30.

### Tensioactif(s) anionique(s) (A) comportant dans leur structure un ou plusieurs groupes sulfate et/ou sulfonate et/ou phosphate.

De préférence, le ou les tensioactifs (A) utilisés selon l'invention ne comprennent pas dans leur structure de groupes carboxyliques (COOH) ou de groupes carboxyliques sous forme de sel (COO⁻).

Parmi les tensioactifs (A), on préfère utiliser les tensioactifs anioniques comportant dans leur structure au moins un groupe choisi parmi les sulfates et les sulfonates.

Le ou les tensioactifs (A) peuvent être oxyéthylénés et/ou oxyproprylénés. Le nombre moyen total de groupements oxyde d'éthylène (OE) et/ou oxyde de propylène (OP) peut alors varier de 2 à 50 et notamment de 2 à 10.

Le ou les tensioactifs (A) utilisables selon l'invention, seul(s) ou en mélange(s), peuvent être choisis parmi les alkylsulfates, les alkylamidosulfates, les alkyléthersulfates, les alkylamidoéthersulfates, alkylaryléthersulfates, les alkyléthersulfosuccinates, les acyl iséthionates, les méthyl acyl taurates, et leurs sels ; le groupe alkyle ou acyle de tous ces différents composés comportant de préférence de 8 à 24 atomes de carbone, et le groupe aryle désignant de préférence un groupement phényle ou benzyle.

De préférence, le ou les tensioactifs (A) sont sous forme de sels, et en particulier de sels alcalins notamment de sels de sodium, de sels d'ammonium, de sels d'amines dont les sels d'aminoalcools, et/ou de sels de magnésium.

En particulier, parmi les tensioactifs anioniques (A) comportant dans leur structure un ou plusieurs groupes sulfate et/ou sulfonate, on préfère encore plus utiliser ceux choisis parmi les alkylsulfates et les alkyléthersulfates, en C₈-C₁₄ et plus particulièrement ceux en C₁₂-C₁₄. Ces sels comprennent de préférence de 2 à 5 groupements d'oxyde d'éthylène. On utilise de manière plus préférée parmi ceux-ci, seuls ou en mélange(s), les alkyl(C₁₂-C₁₄)sulfates de sodium, de triéthanolamine, de magnésium ou d'ammonium, et/ou les alkyl(C₁₂-C₁₄)éthersulfates de sodium, d'ammonium ou de magnésium, oxyéthylénés, par exemple à 2,2 moles d'oxyde d'éthylène. Mieux encore, le ou les tensioactifs (A) sont choisis parmi les alkyl(C₁₂-C₁₄)éthersulfates de sodium, d'ammonium ou de magnésium, oxyéthylénés à 2,2 moles d'oxyde d'éthylène, tels que commercialisés sous la dénomination TEXAPON N702 par la société COGNIS.

Le ou les tensioactifs (A) sont présents à raison de préférence de 1 % à 50 % en poids, en particulier de 2 % à 25 % en poids, plus préférentiellement de 3 % à 20 % en poids, et mieux encore de 3,5 % à 8 % en poids, par rapport au poids total de la composition.

### Tensioactif(s) anionique(s) carboxylique(s) (B)

Selon l'invention, le ou les tensioactifs anioniques carboxyliques sont des tensioactifs anioniques comportant dans leur structure au moins une fonction carboxylique (-COOH) éventuellement sous forme de sel d'acide carboxylique (-COO⁻).

Le ou les tensioactifs anioniques carboxyliques utilisés selon l'invention sont différents des tensioactifs (A) et ne comprennent de préférence pas de groupes sulfate, sulfonate et/ou phosphate.

Le ou les tensioactifs (B) utilisés dans le cadre de la présente invention peuvent être choisis, seul(s) ou en mélange(s), parmi :
- les acides alkyl(C₆-C₂₄)-D-galactoside uroniques et leurs sels,
- les acides acyl(C₆-C₂₄) sarcosiniques et leurs sels,
- les acides acyl(C₆-C₂₄) lactyliques et leurs sels,
- les acides acyl(C₆-C₂₄) glutamiques et leurs sels.

On peut également utiliser les acides alkyl(poly)glycoside carboxyliques, préférentiellement sous forme de sels, tels que les acétates d'alkylglucoside, les citrates d'alkylglucoside et les tartrates d'alkylpolyglycoside ; le groupe alkyle de ces acides alkyl(poly)glycoside carboxyliques comportant de préférence de 6 à 24 atomes de carbone. De tels produits sont notamment vendus sous les dénominations de EUCAROL APG/EC et EUCAROL APG/ET par la société LAMBERTI et PLANTAPON LGC SORB par la société COGNIS.

Le ou les tensioactifs (B) utilisés dans le cadre de la présente invention peuvent être oxyalkylénés, et de préférence oxyéthylénés et/ou oxypropylénés. Le nombre moyen total de groupements oxyde d'alkylène varie alors de préférence de 2 à 50, en particulier de 2 à 24, et mieux encore de 2 à 15.

Lorsque le ou les tensioactifs (B) sont oxyalkylénés, ils peuvent de préférence être choisis, seul(s) ou en mélange(s), parmi :
- les acides alkyl(C₆-C₂₄) éther carboxyliques polyoxyalkylénés,
- les acides alkyl(C₆-C₂₄) aryl éther carboxyliques polyoxyalkylénés,
- les acides alkyl(C₆-C₂₄) amido éther carboxyliques polyoxyalkylénés,
- et leurs sels ;
et en particulier, ceux comportant de 2 à 50 groupements oxyde d'alkylène, de préférence de 2 à 50 groupements oxyde d'éthylène (OE), et mieux encore de 2 à 15 groupements oxyde d'éthylène.

Les sels sont en particulier choisis parmi les sels alcalins notamment de sodium, les sels d'ammonium, les sels d'amines dont les sels d'aminoalcools tels que les sels de triéthanolamine ou de monoéthanolamine, et les sels de magnésium.

On utilise de préférence seuls ou en mélanges :
- les acides acyl(C₆-C₂₄) glutamiques et leurs sels ;
- les acides alkyl(C₆-C₂₄) polyglycoside carboxyliques et leurs sels ;
- les acides alkyl(C₆-C₂₄) éther carboxyliques polyoxyalkylénés et leurs sels, en particulier, ceux comportant de 2 à 24 groupements oxyde d'alkylène, de préférence de 2 à 24 groupements oxyde d'éthylène, et mieux encore de 2 à 15 groupements oxyde d'éthylène ;
- les acides alkyl(C₆-C₂₄) aryl éther carboxyliques polyoxyalkylénés et leurs sels, en particulier, ceux comportant de 2 à 24 groupements oxyde d'alkylène, de préférence de 2 à 24 groupements oxyde d'éthylène, et mieux encore de 2 à 15 groupements oxyde d'éthylène ;
- les acides alkyl(C₆-C₂₄) amido éther carboxyliques polyoxyalkylénés et leurs sels, en particulier, ceux comportant de 2 à 24 groupements oxyde d'alkylène, de préférence de 2 à 24 groupements oxyde d'éthylène, et mieux encore de 2 à 15 groupements oxyde d'éthylène.

De manière plus préférée, on utilise les tensioactifs anioniques carboxyliques polyéthoxylés qui répondent à la formule (I) suivante :

R₁(OC₂H₄)ₙOCH₂COOA (I)

dans laquelle :
R₁ représente un groupe ou un mélange de groupes alkyle ou alcényle, linéaire ou ramifié, en C₈-C₂₂, un groupe alkyl(C₈-C₉) phényle, un groupe R₂CONH-CH₂-CH₂- avec R₂ désignant un groupe alkyle ou alcényle, linéaire ou ramifié, en C₁₁-C₂₁,
n est un nombre entier ou décimal (valeur moyenne) pouvant varier de 2 à 24, et de préférence de 2 à 10,
A désigne H, NH₄, Na, K, Li, Mg ou un reste monoéthanolamine ou triéthanolamine. On peut également utiliser des mélanges de composés de formule (I) en particulier des mélanges dans lesquels les groupements R₁ sont différents.

De préférence, R₁ désigne un groupe ou un mélange de groupes choisis parmi les groupes alkyle en C₁₂-C₁₄, cocoyl, oléyl, nonylphényle, et octylphényle ; A désigne un atome d'hydrogène ou de sodium ; et n varie de 2 à 20, et de préférence 2 à 10.

Plus préférentiellement encore, on utilise des composés de formule (I) dans laquelle R₁ désigne un groupe alkyle en C₁₂ ; A désigne un atome d'hydrogène ou de sodium ; et n varie de 2 à 10.

Parmi les produits commerciaux, on peut utiliser de préférence les produits vendus par la société CHEM Y sous les dénominations :
AKYPO® NP 70 (R₁=nonylphényle, n=7, A=H)
AKYPO® NP 40 (R₁=nonylphényle, n=4, A=H)
AKYPO®OP 40 (R₁=octylphényle, n=4, A=H)
AKYPO® OP 80 (R₁=octylphényle, n=8, A=H)
AKYPO® OP 190 (R₁=octylphényle, n=19, A=H)
AKYPO® RLM 38 (R₁= alkyle en C₁₂-C₁₄, n=4, A=H)
AKYPO® RLM 38 NV (R₁= alkyle en C₁₂-C₁₄, n=4, A=Na)
AKYPO® RLM 45 CA (R₁ = alkyle en C₁₂-C₁₄, n=4,5, A=H)
AKYPO® RLM 45 NV (R₁= alkyle en C₁₂-C₁₄, n=4,5, A=Na)
AKYPO® RLM 100 (R₁= alkyle en C₁₂-C₁₄, n=10, A=H)
AKYPO® RLM 100 NV (R₁= alkyle en C₁₂-C₁₄, n=10, A=Na)
AKYPO® RLM 130 (R₁= alkyle en C₁₂-C₁₄, n=13, A=H)
AKYPO® RLM 160 NV (R₁= alkyle en C₁₂-C₁₄, n=16, A=Na),
ou par la société SANDOZ sous les dénominations :
SANDOPAN DTC-Acid (R₁= alkyle en C₁₃, n=6, A=H)
SANDOPAN DTC (R₁= alkyle en C₁₃, n=6, A=Na)
SANDOPAN LS 24 (R₁= alkyle en C₁₂-C₁₄, n=12, A=Na)
SANDOPAN JA 36 (R₁= alkyle en C₁₃, n=18, A=H),
et plus particulièrement, les produits vendus sous les dénominations suivantes :
AKYPO® RLM 45 (INCI : Laureth-5 carboxylic acid)
AKYPO®RLM 100
AKYPO® RLM 38.

Le ou les tensioactifs (B) sont présents à raison de préférence de 0,5 % à 15 % en poids, en particulier de 1 % à 10 % en poids, et mieux encore de 1,5 % à 8 % en poids, par rapport au poids total de la composition.

### Tensioactif amphotère(s) et/ou zwittérionique(s) (C)

Le ou les tensioactifs amphotères et/ou zwittérioniques peuvent être notamment (liste non limitative) des dérivés d'amines secondaires ou tertiaires aliphatiques, dans lesquels le ou les substituants aliphatiques de la fonction amine secondaire ou tertiaire sont choisis parmi les chaînes linéaires ou ramifiées comportant 8 à 22 atomes de carbone et contenant au moins un groupe anionique hydrosolubilisant tel que par exemple un groupe carboxylate, sulfonate, sulfate, phosphate et/ou phosphonate ; et/ou des bétaïnes, telles que celles citées ci-après.

Parmi les dérivés d'amines utilisables dans le cadre de la présente invention, on peut citer, seuls ou en mélanges, les produits décrits dans les brevets US-2 528 378 et US-2 781 354 et répondant à l'une des structures de formule (II) ou (III) suivante :

- formule (II) : R₂ -CONHCH₂CH₂-N⁺ (R₃)(R₄)(CH₂COC⁻) (II)

dans laquelle :
R₂-CO désigne un groupe ou un mélange de groupes choisis parmi les groupes acyle en C₆-C₂₄, par exemple la partie acyle en C₆-C₂₄ correspondant à un des acides gras de formule R₂-COOH présents dans l'huile de coprah hydrolysée, un groupe octoyle, un groupe décoyle, un groupe dodécanoyle, ou un mélange de ces groupes,
R₃ désigne un groupement bêta-hydroxyéthyle,
R₄ désigne un groupement carboxyméthyle.

- formule (III) : R₂-CONHCH₂CH₂-N(B)(C) (III)

dans laquelle :
B représente -CH₂CH₂OX',
C représente -(CH₂)_{z} -Y', avec z = 1 ou 2,
X' désigne le groupement -CH₂CH₂-COOH ou un atome d'hydrogène,
Y' désigne -COOH ou le groupe -CH₂-CHOH-SO₃H,
R₂-CO désigne un groupe acyle en C₆-C₂₄, par exemple la partie acyle en C₆-C₂₄ correspondant à un des acides gras de formule R₂-COOH présents dans l'huile de coprah hydrolysée ou l'huile de lin, un groupe octoyle, un groupe décoyle, un groupe dodécanoyle, un groupe stéaroyle, un groupe isostéaroyle, un groupe oléoyle, ou un mélange de ces groupes.

Ces composés sont classés dans le dictionnaire CTFA, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple, on peut citer le Disodium Cocoamphodiacetate commercialisé sous la dénomination commerciale MIRANOL® C2M concentré par la société RHODIA CHIMIE.

Parmi les bétaïnes utilisables dans le cadre de la présente invention, on peut citer les alkyl(C₈-C₂₀) bétaïnes, les sulfobétaïnes, les alkyl(C₈-C₂₀) amidoalkyl(C₁-C₆) bétaïnes et les alkyl(C₈-C₂₀) amidoalkyl(C₁-C₆) sulfobétaïnes, éventuellement hydroxylées, seules ou en mélanges.

De préférence, le ou les tensioactifs amphotériques et/ou zwittérioniques employés dans la présente invention sont choisis parmi les bétaïnes ci-dessus et leurs mélanges. On préfère plus particulièrement utiliser les alkyl(C₈-C₂₀) bétaïnes en particulier la cocobétaïne commercialisée par la société COGNIS sous la dénomination DEHYTON AB 30 en solution aqueuse à 30 % en poids de Matière Active (MA) par rapport au poids total de la solution ; les alkyl(C₈-C₂₀) amidoalkyl(C₁-C₆) bétaïnes, en particulier la cocamidopropylbétaine telle que commercialisée sous la dénomination TEGOBETAINE® F50 par la société GOLDSCHMIDT.

Avantageusement, le ou les tensioactif(s) (C) sont choisis parmi les bétaïnes.

Le ou les tensioactifs (C) sont présents dans des quantités allant de préférence de 0,1 % à 20 % en poids, en particulier de 1 % à 15 % en poids, et mieux encore de 2 % à 10 % en poids, par rapport au poids total de la composition.

### Tensioactif(s) non ionique(s) alkyl(poly)glycoside (D)

Par "alky(poly)glycoside", on désigne un alkylpolyglycoside ou un alkylmonoglycoside aussi appelé alkylglycoside dans la présente demande, pouvant être alkoxylé par un ou plusieurs groupes oxyde d'alkylène préférentiellement en C₂-C₄.

Le ou les tensioactifs non ioniques alkyl(poly)glycoside utilisés, seul(s) ou en mélange(s), conformément à la présente invention peuvent être représentés par la formule (IV) suivante :

R₁O-(R₂O)ₜ (G)ᵥ (IV)

dans laquelle :
R₁ représente un groupe alkyle saturé ou insaturé, linéaire ou ramifié, comportant environ de 8 à 24 atomes de carbone, un groupe alkylphényle dont le groupe alkyle linéaire ou ramifié comporte de 8 à 24 atomes de carbone,
R₂ représente un groupe alkylène comportant environ de 2 à 4 atomes de carbone,
G représente un motif saccharidique comportant de 5 à 6 atomes de carbone,
t désigne une valeur allant de 0 à 10, de préférence 0 à 4, et
v désigne une valeur allant de 1 à 15.

De préférence, le ou les tensioactifs non ioniques alkyl(poly)glycoside répondent à la formule (IV) dans laquelle :
R₁ désigne un groupe alkyle saturé ou insaturé, linéaire ou ramifié, comportant de 8 à 18 atomes de carbone,
G désigne le glucose, le fructose ou le galactose, et de préférence le glucose,
t désigne une valeur allant de 0 à 3, et est de préférence égale à 0,
et R₂ et v sont tels que définis précédemment.

Le degré de polymérisation du ou des tensioactifs non ioniques alkyl(poly)glycoside, tel que représenté par exemple par l'indice v dans la formule (IV), varie en moyenne de 1 à 1,5, et de préférence de 1 à 4. Ce degré de polymérisation varie plus particulièrement de 1 à 2, et mieux encore de 1,1 à 1,5, en moyenne.

Les liaisons glycosidiques entre les motifs saccharidiques sont en 1,6 ou en 1,4 ; et de préférence en 1,4.

Les composés de formule (IV) pouvant être utilisés dans la présente invention sont notamment représentés par les produits vendus par la société COGNIS sous les dénominations PLANTAREN® (600 CS/U, 1200 et 2000) ou PLANTACARE® (818, 1200 et 2000). On peut également utiliser les produits vendus par la société SEPPIC sous les dénominations TRITON CG 110 (ou ORAMIX CG 110) et TRITON CG 312 (ou ORAMIX® NS 10), les produits vendus par la société B.A.S.F. sous la dénomination LUTENSOL GD 70 ou encore ceux vendus par la société CHEM Y sous la dénomination AG10 LK.

On peut également utiliser l'alkyl(C₈-C₁₆) polyglucoside en 1,4 par exemple en solution aqueuse à 53 % en poids du poids total de la solution, commercialisé par COGNIS sous la référence PLANTACARE® 818 UP.

Le ou les tensioactifs (D) sont présents dans des quantités allant de préférence de 0,1 % à 20 % en poids, et en particulier de 1 % à 15 % en poids, par rapport au poids total de la composition.

La quantité minimale de tensioactifs anionique(s), amphotérique(s) et/ou zwittérionique(s), et non ionique(s) est celle suffisante pour conférer à la composition un pouvoir moussant et/ou détergent satisfaisant. Des quantités trop importantes de tensioactifs n'apportent pas vraiment d'avantages supplémentaires.

Ainsi, selon l'invention, la quantité totale de tensioactifs anionique(s), amphotérique(s) et/ou zwittérionique(s), et non ionique(s) peut représenter de 4 % à 50 % en poids, de préférence de 6 % à 35 % en poids, et en particulier de 8 % à 25 % en poids, du poids total de la composition.

Le rapport pondéral de la quantité de tensioactif(s) (A) sur la quantité de tensioactif(s) (B) va de préférence de 0,1 à 10, et en particulier de 0,5 à 5.

Le rapport pondéral de la quantité de tensioactif(s) (A) sur la quantité de tensioactif(s) (C) va de préférence de 0,1 à 10, en particulier de 0,5 à 5, et mieux encore de 0,7 à 2.

Le rapport pondéral de la quantité de tensioactif(s) (A) sur la quantité de tensioactif(s) (D) va de préférence de 0,1 à 10, en particulier de 0,2 à 5, et mieux encore de 0,5 à 2.

Le rapport pondéral de la quantité de tensioactif(s) anionique(s) (B) sur la quantité de tensioactif(s) (C) va de préférence de 0,1 à 10, en particulier de 0,2 à 5, et mieux encore de 0,2 à 5.

### Polymère(s) cationique(s) (E)

Le ou les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, FR-A-2 383 660, FR-A-2 598 611, FR-A-2 470 596, FR-A-2 519 863 et FR-A-2875 503.

Le ou les polymères cationiques préférés sont choisis parmi ceux qui contiennent dans leur structure des motifs comportant des groupements amine primaires, secondaires, tertiaires et/ou quaternaires pouvant par exemple soit faire partie de la chaîne principale polymère, soit être portés par un substituant latéral directement relié à celle-ci.

Parmi les polymères cationiques, on peut citer plus particulièrement les polymères de la famille des polyamines, polyaminoamides et polyammoniums quaternaires. Parmi ces polymères, on peut citer :
(1) Les homopolymères ou copolymères dérivés d'esters ou d'amides acryliques ou méthacryliques, réticulés ou non, et comportant au moins un des motifs de formules (V), (VI), (VII) ou (VIII) suivantes : 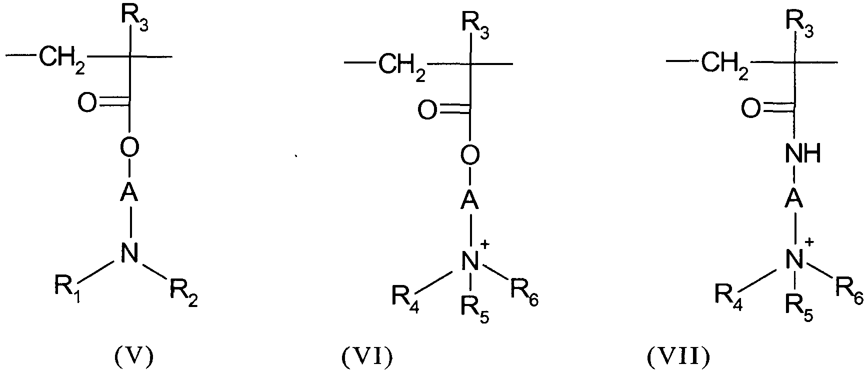 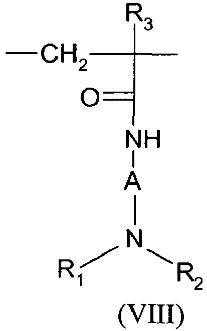 dans lesquelles
   R₁ et R₂, identiques ou différents, représentent chacun un atome d'hydrogène ou un groupe alkyle ayant de 1 à 6 atomes de carbone et de préférence méthyle ou éthyle ;
   R₃, identiques ou différents, désignent chacun un atome d'hydrogène ou un groupe CH₃ ;
   A, identiques ou différents, représentent chacun un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent chacun un groupe alkyle ayant de 1 à 6 atomes de carbone ou un groupe benzyle et de préférence un groupe alkyle ayant de 1 à 6 atomes de carbone ;
   X- désigne un anion dérivé d'un acide minéral ou organique tel qu'un anion méthosulfate ou un halogénure tel que chlorure ou bromure.

   Les polymères de la famille (1) peuvent contenir en outre un ou plusieurs motif(s) dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle / vinylcaprolactame / vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - les copolymères vinylpyrrolidone / méthacrylamide de diméthylaminopropyle quaternisés tel que le produit vendu sous la dénomination "GAFQUAT HS 100" par la société ISP,
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide / chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et "SALCARE® SC 96 " par la Société CIBA.
(2) Les polysaccharides cationiques notamment choisis parmi :
   a) les dérivés d'éther de cellulose comportant des groupements ammonium quaternaire décrits dans le brevet français 1 492 597, et en particulier les polymères commercialisés sous les dénominations « JR » (JR 400, JR 125, JR 30M) ou « LR » (LR 400, LR 30M) par la société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
   b) les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl-celluloses greffées notamment avec un sel de méthacryloyléthyl triméthylammonium, de méthacrylmidopropyl triméthylammonium ou de diméthyl-diallylammonium.
      Les produits commercialisés répondant à cette définition sont plus particulièrement les produits vendus sous la dénomination « Celquat L 200 » et « Celquat H 100 » par la société National Starch.
   c) les gommes de guar contenant des groupements cationiques trialkylammonium. On utilise par exemple des gommes de guar modifiées par un sel (par exemple, un sel de chlorure) de 2,3-époxypropyl triméthylammonium.
      De tels produits sont commercialisés notamment sous les dénominations commerciales de JAGUAR C 13 S, JAGUAR C 15, JAGUAR C 17 ou JAGUAR C162 par la société MEYHALL
(3) Les polymères constitués de motifs pipérazinyle et de radicaux divalents alkylène ou hydroxyalkylène à chaîne droite ou ramifiée, éventuellement interrompue par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(4) Les polyaminoamides cationiques solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; ces polyaminoamides peuvent être alcoylés ou s'ils comportent une ou plusieurs fonctions amines tertiaires, quaternisées. De tels polymères sont notamment décrits dans les brevets français 2 252 840 et 2 368 508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylamino hydroxyalcoyldialcoylène triamine dans lesquels le groupe alcoyle comporte de 1 à 4 atomes de carbone et désigne de préférence méthyle, éthyle, propyle. De tels polymères sont notamment décrits dans le brevet français 1 583 363.
   Parmi ces dérivés, on peut citer plus particulièrement les polymères acide adipique / diméthylaminohydroxypropyl / diéthylène triamine vendus sous la dénomination "Cartaretine F, F4 ou F8" par la société Sandoz.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique / époxypropyl / diéthylène-triamine.
(7) Les cyclopolymères d'alkyldiallylamine ou de dialkyldiallylammonium tels que les homopolymères ou copolymères comportant, comme constituant principal de la chaîne, des motifs répondant aux formules (IX) ou (X) : 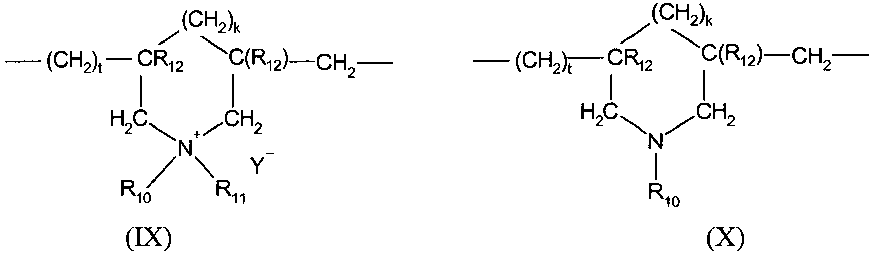 dans lesquelles k et t sont égaux à 0 ou 1, la somme k + t étant égale à 1 ; R₁₂ désigne un atome d'hydrogène ou un groupe méthyle ; R₁₀ et R₁₁, désignent chacun, indépendamment l'un de l'autre, un groupement alkyle ayant de 1 à 6 atomes de carbone, un groupement hydroxyalkyle dans lequel le groupement alkyle a de préférence de 1 à 5 atomes de carbone, un groupement amidoalkyle inférieur (i.e. dont la partie alkyle est en C₁-C₄), ou alors R₁₀ et R₁₁ peuvent désigner conjointement avec l'atome d'azote auquel ils sont rattachés, un groupement hétérocyclique, tel que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   De préférence, R₁₀ et R₁₁ désignent chacun, indépendamment l'un de l'autre, un groupement alkyle ayant de 1 à 4 atomes de carbone.
   Parmi les polymères définis ci-dessus, on peut citer les homopolymères de chlorure de dialkyldiallylammonium, plus particulièrement l'homopolymère de chlorure de diméthyldiallylammonium (nom INCI : Polyquaternium-6) vendu sous la dénomination "MERQUAT® 100" par la société NALCO (et ses homologues de faibles masses moléculaires moyenne en poids) et les copolymères de chlorure de dialkyldiallylammonium, plus particulièrement le copolymère de chlorure de diméthyldiallylammonium et d'acrylamide commercialisés sous la dénomination "MERQUAT® 550".
(8) Les polymères de diammonium quaternaire contenant des motifs récurrents répondant à la formule (XI) : 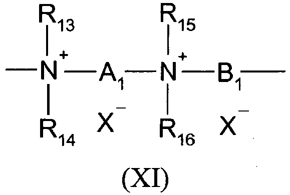 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs (i.e. dont la partie alkyle est en C₁-C₄), ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent chacun un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique ; A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)ₙ-
   dans lequel n désigne un nombre entier de 0 à 7 et E' désigne :
   a) un reste de glycol de formule -O-Z-O-, où Z désigne un groupe hydrocarboné linéaire ou ramifié, ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent chacun un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
   d) un groupement uréylène de formule -NH-CO-NH-.

   De préférence, X- est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule (XII) : 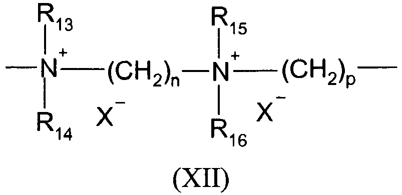 dans laquelle R₁₃, R₁₄, R₁₅ et R₁₆, identiques ou différents, désignent chacun un groupe alkyle ou hydroxyalkyle ayant de 1 à 4 atomes de carbone environ, n et p sont des nombres entiers variant de 2 à 20 environ et, X- est un anion dérivé d'un acide minéral ou organique. De préférence, R₁₃, R₁₄, R₁₅ et R₁₆ désignent chacun un groupe méthyle. A titre d'exemple de polymère utilisable répondant à la formule (XII), on peut citer le chlorure d'hexadiméthrine, commercialisé sous la dénomination MEXOMERE PO par la société CHIMEX.
(9) Les polymères de polyammonium quaternaire constitués de motifs de formule (XIII) : 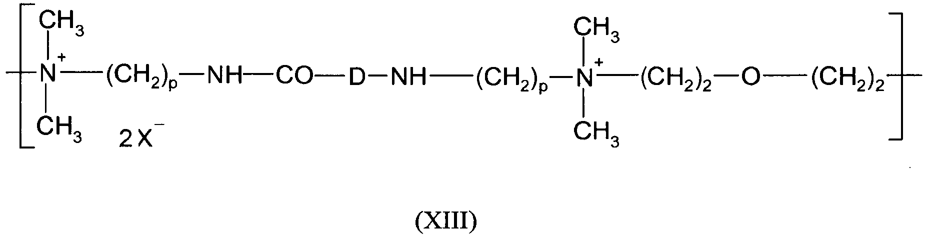 dans laquelle :
   p désigne un nombre entier variant de 1 à 6 environ,
   D peut être nul ou peut représenter un groupement
   -(CH₂)ᵣ -CO- dans lequel r désigne un nombre égal à 4 ou à 7, et
   X- est un anion dérivé d'un acide minéral ou organique.
      Les polymères cationiques comportant des motifs de formule (XIII) sont notamment décrits dans la demande de brevet EP-A-122 324 et peuvent être préparés selon les procédés décrits dans les brevets U.S.A. n° 4 157 388, 4 390 689, 4 702 906, 4 719 282.
      Parmi ces polymères, on préfère ceux de masse moléculaire mesurée par RMN du Carbone 13 inférieure à 100000, et dans la formule de laquelle :
      p est égal à 3, et,
         a) D représente un groupement -(CH₂)₄-CO- , X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1,
         b) D représente un groupement -(CH₂)₇-CO-, X désigne un atome de chlore, la masse moléculaire mesurée par RMN du Carbone 13 (RMN¹³C) étant d'environ 8100 ; un polymère de ce type est proposé par la société MIRANOL sous le nom de MIRAPOL-AZ1,
         c) D désigne la valeur zéro, X désigne un atome de chlore, la masse moléculaire mesurée par RMN du Carbone 13, (RMN¹³C) étant d'environ 25500 ; un polymère de ce type est vendu par la société MIRANOL sous le nom MIRAPOL-A15,
         d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).

         Plus particulièrement encore, on préfère selon l'invention le polymère à motifs de formule (XIII) dans laquelle p est égal à 3, D désigne la valeur zéro, X désigne un atome de chlore, la masse moléculaire mesurée par RMN du Carbone 13, (RMN¹³C) étant d'environ 25500.
(10) Les polymères quaternaires de vinylpyrrolidone et de vinylimidazole tels que par exemple les produits commercialisés sous les dénominations Luviquat FC 905, FC 550 et FC 370 par la société B.A.S.F.
(11) Les polyamines cationiques comme le Polyquart H vendu par HENKEL, référencé sous le nom de " POLYETHYLENEGLYCOL (15) TALLOW POLYAMINE " dans le dictionnaire CTFA.
(12) Les homopolymères ou copolymères de vinylamide et en particulier les homopolymères de vinylamide partiellement hydrolysés tels que les poly(vinylamine/vinylamide). Ces polymères sont formés à partir d'au moins un monomère vinylamide répondant à la formule suivante :

   H₂C=CR²NRC(O)R¹

   dans laquelle R, R¹ et R² sont chacun choisis parmi un atome d'hydrogène, un groupe alkyle en C₁-C₂₀, un groupe aryle et un groupe alkylaryle dont la partie alkyle comprend de 1 à 20 atomes de carbone.
   En particulier, ledit monomère peut être choisi parmi le N-vinylformamide, le N-méthyl-N-vinylacétamide et le N-vinyl acétamide. De préférence, on utilise le poly(vinylamine/N-vinylformamide) tel que commercialisé sous la dénomination CATIOFAST VMP par la société BASF ou sous la dénomination LUPAMIN 9030 par la société BASF.
   Ces polymères peuvent être formés par exemple par polymérisation radicalaire d'un monomère vinylamide puis hydrolyse acide ou basique partielle des fonctions amides en fonctions amines quaternisables, tel que décrit dans les demandes WO 2007/005577, US 5,374,334, US 6,426,383 et US 6,894,110.
(13) Les polyuréthanes constitués essentiellement :
   (a1) d'au moins un motif cationique dérivé d'au moins une amine tertiaire ou quaternaire présentant au moins deux fonctions réactives à hydrogène labile,
   (a2) d'au moins un motif non ionique dérivé d'au moins une polyoléfine présentant au moins deux fonctions réactives à hydrogène labile, ladite polyoléfine comprenant au moins 10 % en moles d'unités comprenant au moins une double liaison C=C (carbone-carbone), par rapport à la totalité des unités formant ladite polyoléfine ;
   (b) d'au moins un motif dérivé d'un composé comportant au moins deux fonctions isocyanate.

Le polymère selon l'invention présente de préférence un caractère élastique ; on entend par là que ledit polymère est un matériau macromoléculaire qui retourne rapidement à sa forme et à ses dimensions initiales après cessation d'une contrainte faible ayant produit une déformation importante.

Ces polymères peuvent être obtenus par polycondensation des composés portant des fonctions réactives à hydrogène labile avec des composés comportant au moins deux fonctions isocyanate ;

On entend par fonctions réactives à hydrogène labile des fonctions capables, après départ d'un atome d'hydrogène, de former des liaisons covalentes avec les fonctions isocyanate des composés comportant au moins deux fonctions isocyanate. On peut citer à titre d'exemple de telles fonctions les groupes hydroxyle, amine primaire, amine secondaire, ou encore les groupes thiol.

Selon la nature des fonctions réactives portant l'hydrogène labile (-OH, -NH₂, -NHR ou -SH), la polycondensation conduit à, respectivement, des polyuréthanes, des polyurées ou des polythiouréthanes. Ainsi, les polymères utilisables dans les compositions selon l'invention peuvent être des copolymères uréthane/urée et/ou thiouréthane. Tous ces polymères sont regroupés dans la présente demande, par souci de simplification, sous le terme de polyuréthanes.

Le ou les polyuréthanes cationiques utilisables dans la composition selon l'invention comprennent donc au moins un motif cationique (a1) résultant d'au moins une amine tertiaire ou quaternaire présentant au moins deux fonctions réactives à hydrogène labile.

L'amine tertiaire est de préférence protonable à un pH choisi entre pH 1 et pH 12. Par "protonable", on entend que ladite fonction amine tertiaire peut être neutralisée au moins partiellement par un agent neutralisant ou par une fonction du milieu dans lequel il est formulé.

Lorsque les amines tertiaires ou quaternaires formant les motifs (a1) portent plus de deux fonctions à hydrogène labile, les polyuréthanes obtenus présentent une structure ramifiée.

Toutefois, de préférence, les amines tertiaires ou quaternaires formant les motifs (a1) ne présentent que deux fonctions réactives à hydrogène labile et les polyuréthanes obtenus par polycondensation ont par conséquent une structure essentiellement linéaire.

Il est bien entendu également possible d'utiliser un mélange d'amines difonctionnelles contenant, ou non, une faible proportion d'amines portant plus de deux fonctions réactives à hydrogène labile.

Les amines tertiaires ou quaternaires formant les motifs cationiques (a1) sont de préférence choisies parmi les composés correspondant à l'une ou plusieurs des formules suivantes : 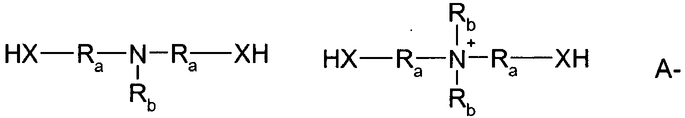 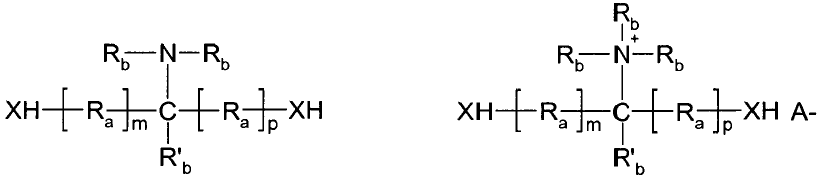 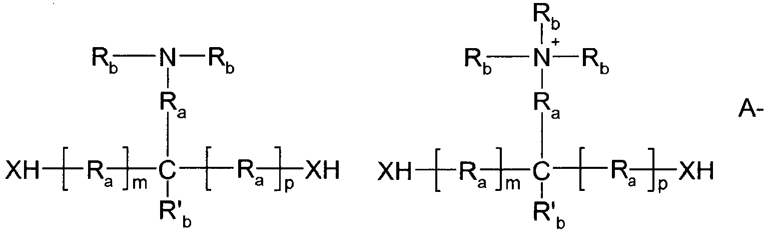 dans lesquelles :
- chaque Rₐ, indépendamment les uns des autres, représente un groupe divalent alkylène en C₁-C₆, linéaire ou ramifié, ou bien cycloalkylène en C₃-C₆ ou arylène, ou leurs mélanges ; ces groupes pouvant être substitués par un ou plusieurs atomes d'halogène et/ou comporter un ou plusieurs hétéroatomes choisis parmi O, N, P et S,
- chaque R_{b} représente indépendamment les uns des autres, un groupe alkyle linéaire ou ramifié, en C₁-C₆, ou bien cycloalkyle en C₃-C₆ ou encore aryle, ou leurs mélanges ; ces groupes pouvant être substitués par un ou plusieurs atomes d'halogène et/ou comporter un ou plusieurs hétéroatomes choisis parmi O, N, P et S,
- chaque R'_{b} représente un atome d'hydrogène ou un groupe alkyle linéaire ou ramifié, en C₁-C₆, ou bien cycloalkyle en C₃-C₆ ou encore aryle, ou leurs mélanges ; ces groupes pouvant être substitués par un ou plusieurs atomes d'halogène et/ou comporter un ou plusieurs hétéroatomes choisis parmi O, N, P et S,
- m et p sont, indépendamment l'un de l'autre, égaux à 0 ou 1 ; de préférence m= 1 et p= 1 ;
- chaque X représente, indépendamment les uns des autres, un atome d'oxygène ou de soufre, ou un groupe NH ou NR_{c}, où R_{c} représente un groupe alkyle en C₁-C₆, et
- A⁻ représente un contre-ion physiologiquement acceptable, et notamment un halogénure tel que chlorure ou bromure.

De préférence, les amines sont choisies parmi les composés répondant à l'une ou plusieurs des formules : 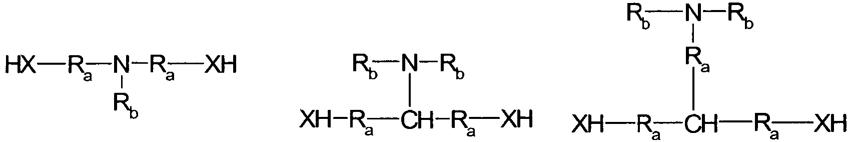 dans lesquelles :
- Rₐ est un groupe divalent alkylène en C₁-C₆, linéaire ou ramifié, notamment méthylène ou éthylène ; et/ou
- R_{b} est un groupe alkyle linéaire ou ramifié, en C₁-C₆, notamment un groupe méthyle, éthyle, n-butyle, isobutyle ou tert-butyle ; et/ou
- X désigne un atome d'oxygène.

Encore plus préférentiellement, les amines sont de formule : 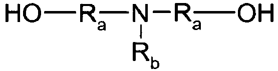 dans laquelle Rₐ est un groupe divalent alkylène en C₁-C₆, linéaire ou ramifié, notamment méthylène ou éthylène ; et R_{b} est un groupe alkyle linéaire ou ramifié, en C₁-C₆, notamment un groupe méthyle, éthyle, n-butyle, isobutyle ou tert-butyle.

On peut citer à titre d'amines tertiaires particulièrement préférées, la N-méthyldiéthanolamine et la N-tert-butyldiéthanolamine.

Les amines tertiaires, protonables, peuvent être neutralisées, totalement ou partiellement, par un agent neutralisant, de type acide organique comprenant au moins une fonction acide carboxylique, sulfonique et/ou phosphorique ou par un acide minéral. On peut citer à titre d'exemple d'acides préférés l'acide chlorhydrique, l'acide sulfurique, l'acide acétique, l'acide propionique, l'acide citrique, l'acide gluconique, l'acide tartrique, l'acide lactique, l'acide phosphorique, l'acide benzoïque, l'acide stéarique, l'acide oléique, l'acide 2-éthylcaproïque, l'acide béhénique, le chlorhydrate de bétaïne, et leurs mélanges.

Le ou les polyuréthanes cationiques utilisables dans la composition selon l'invention comprennent également au moins un motif non ionique (a2) résultant d'au moins une polyoléfine présentant au moins deux fonctions réactives à hydrogène labile, ladite polyoléfine comprenant au moins 10 % en mole d'unités comprenant au moins une double liaison C=C (résiduelle), par rapport à la totalité des unités formant ladite polyoléfine.

De préférence, la ou les polyoléfines sont non ioniques.

De préférence, les fonctions réactives à hydrogène labile sont situées aux extrémités de la polyoléfine. Notamment, lesdites fonctions réactives à hydrogène labile sont des hydroxydes. De façon préférentielle, le nombre de motifs hydroxyde est proche de, voire égal à, 2.

De préférence encore, la ou les polyoléfines formant le motif (a2) est choisie parmi les homopolymères et/ou copolymères d'oléfines, portant à leurs extrémités des fonctions réactives à hydrogène labile et ayant une température de transition vitreuse (Tg), mesurée par analyse enthalpique différentielle (DSC, differential scanning calorimetry) selon la norme ASTM D3418-97, inférieure à 10°C.

Le ou les polyuréthanes dans la composition selon l'invention peuvent comprendre plusieurs motifs (a2) résultant de plusieurs polyoléfines, identiques ou différentes (mélanges de polyoléfines) ; toutefois, dans ce cas, chacune des polyoléfines comprend au moins 10 % molaire d'unités comprenant au moins une double liaison C=C.

On entend par « unité » comprenant une double liaison C=C, une unité qui comprend au moins une double liaison C=C résiduelle, de préférence une seule double liaison ; il peut s'agir, par exemple d'une unité issue de la polymérisation d'une unité butadiène ou isoprène, toutes formes isomériques incluses (cis ou trans, 1,2 ou 1,4).

La polyoléfine utilisable peut être un homopolymère d'oléfines. On peut par exemple citer les homopolymères de 1,2-butadiène, de 1,4-butadiène ou d'isoprène, et notamment :
- les 1,4-polybutadiène, sous leurs formes cis et trans : 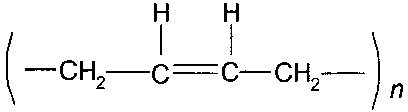
- les 1,2-polybutadiène :

   -[CH₂-CH(CH=CH₂)-]ₙ
- les poly(cis-1,4-isoprène) : 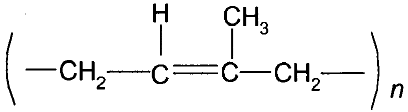
- les poly(trans-1,4-isoprène) :

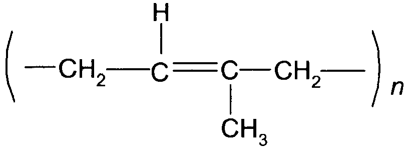

La polyoléfine utilisable peut également être un copolymère de différentes oléfines (copolymère d'oléfines), sous réserve que la polyoléfine finale comprenne au moins 10 % molaire d'unités comprenant au moins une double liaison C=C.

Dans un premier mode de réalisation, ladite polyoléfine peut être exclusivement constituée d'unités comprenant au moins une double liaison C=C. On peut par exemple citer les copolymères, notamment statistiques, comprenant des unités 1,2-butadiène et/ou des unités 1,4-butadiène dans ses formes cis et/ou trans, et/ou des unités isoprène, notamment cis-1,4-isoprène et trans-1,4-isoprène, en mélange. On peut notamment citer les copolymères statistiques (1,2-butadiène/1,4-butadiène).

De préférence, la ou les polyoléfines utilisables peuvent être statistiques et à terminaisons hydroxy et répondre à la structure suivante : 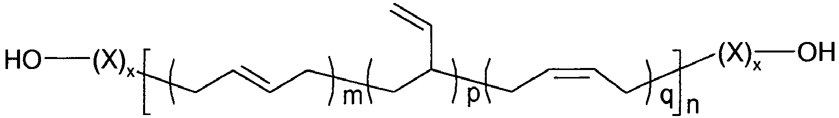 dans laquelle :
m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
n est un entier allant de 10 à 100, notamment de 15 à 50 ;
x=0 ou 1, et
X représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.

Elles peuvent présenter, de préférence, une masse moléculaire moyenne en nombre, Mn, allant de 400 à 50000, de préférence de 500 à 30000, en particulier de 1000 à 15000, et encore mieux de 1500 à 12000.

Plus particulièrement, on peut citer :
- les polybutadiènes à terminaisons hydroxy, tels que les polymères de structure : 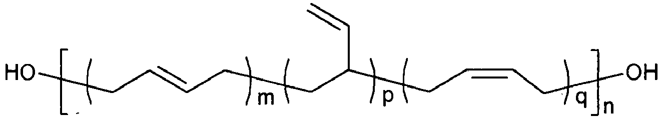 avec m=0,6, p=0,2 et q=0,2 (fractions molaires) et n=25.

On peut en particulier citer les produits commerciaux « Poly bd R20LM » et « Poly bd R45HTLO » de Sartomer.
- les polybutadiènes à terminaisons primaires hydroxy, tels que les polymères pouvant être représentés par la structure suivante : 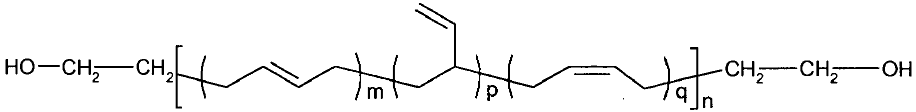 qui sont des copolymères statistiques notamment de 1,4-cis-butadiène et de 1,4-trans-butadiène, avec m=0,17, p=0,65 et q=0,18 (fractions molaires) et n est tel que le poids moléculaire moyen en nombre Mn varie de 1000 à 10000, notamment de 2000 à 6000 (g.mol⁻¹).

On peut en particulier citer les produits commerciaux KRASOL LBH-P 2000, 3000 ou 5000 de Sartomer.
- les polybutadiènes à terminaisons secondaires hydroxy, tels que les polymères pouvant être représentés par la structure suivante : 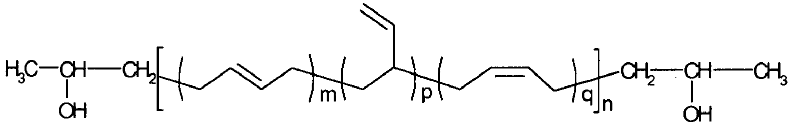 qui sont des copolymères statistiques de 1,4-cis-butadiène et de 1,4-trans-butadiène, avec m=0,17, p=0,65 et q=0,18 (fractions molaires), et n est tel que le poids moléculaire moyen en nombre Mn varie de 1000 à 12000, notamment de 2000 à 10000 (g.mol⁻¹).

On peut en particulier citer les produits commerciaux KRASOL LBH 2000, 3000, 5000 ou 10000 de Sartomer.

Dans un second mode de réalisation, ladite ou lesdites polyoléfines peuvent comprendre en outre des unités additionnelles ne comprenant pas de double liaison C=C.

Toutefois, ces unités additionnelles sont présentes en une quantité maximale de 90 % molaire, étant donné que la polyoléfine finale doit comprendre au moins 10 % molaire d'unités comprenant au moins une double liaison C=C.

Ces unités oléfines additionnelles peuvent notamment être choisies parmi les unités éthylène -(CH₂-CH₂)ₙ-, propylène -(CH₂-CH₂-CH₂)ₙ-, isopropylène -(CH₂CH(CH₃))ₙ-, et/ou butylène de formule : 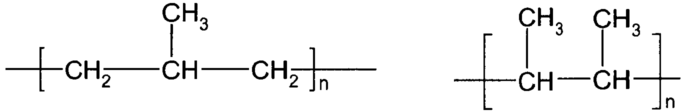 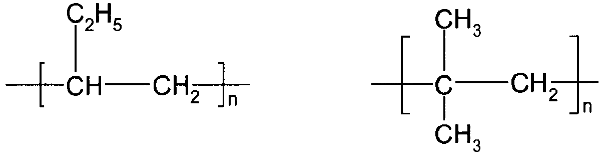 ainsi que leurs mélanges.

Les homopolymères ou copolymères d'oléfines telles que définies ci-dessus peuvent subir, ultérieurement à la polymérisation, une hydrogénation partielle des doubles liaisons résiduelles. Cette hydrogénation ne peut en aucun cas être totale.

En effet, la ou les polyoléfines susceptibles d'être employées pour former les motifs (a2) selon l'invention doivent obligatoirement comprendre au moins 10 % en mole d'unités comprenant au moins une double liaison C=C (résiduelle), par rapport à la totalité des unités formant ladite polyoléfine.

Elles comprennent de préférence au moins 20 % molaire, notamment au moins 40 % molaire, voire au moins 50 % molaire, préférentiellement au moins 80 % molaire, et tout particulièrement 100 % molaire, d'unités comprenant au moins une double liaison C=C, notamment comprenant une seule double liaison C=C.

Cette teneur en unité comportant au moins une double liaison C=C peut notamment être déterminée par les techniques usuelles, notamment par RMN ou par dosage à l'iode.

De préférence, la ou les polyoléfines utilisables pour former les motifs non ioniques (a2) ont une masse moléculaire en nombre (Mn) allant de 400 à 50000, de préférence de 500 à 30000, en particulier de 1000 à 15000, et encore mieux de 1500 à 12000.

De préférence, la ou les polyoléfines susceptibles d'être utilisées dans le cadre de l'invention sont :
- des homopolymères tels que le 1,4-polybutadiène et le 1,2-polybutadiène ;
- des copolymères de structure : 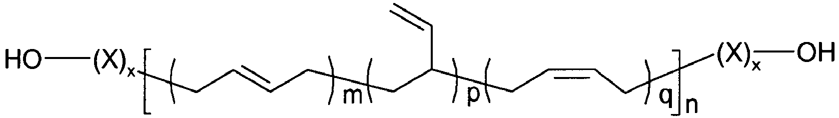 dans laquelle :
   m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
   n est un entier allant de 10 à 100, notamment de 15 à 50 ;
   x=0 ou 1, et
   X représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.

Le ou les polyuréthanes cationiques utilisables dans la composition selon l'invention comprend également au moins un motif (b) résultant d'au moins un composé comportant au moins deux fonctions isocyanate.

Il peut bien évidemment s'agir d'un mélange de plusieurs composés comportant au moins deux fonctions isocyanate.

Les composés comportant au moins deux fonctions isocyanate peuvent être choisis parmi les diisocyanates, ou les mélanges d'un diisocyanate et d'un polyisocyanate comportant plus de deux fonctions isocyanates, ledit polyisocyanate représentant de préférence 0,1 % à 40 % en poids dudit mélange, notamment 0,5 % à 35 % en poids, voire 1 % à 30 % en poids, du poids dudit mélange.

Les composés comportant au moins deux fonctions isocyanate peuvent de préférence être choisis parmi les diisocyanates aliphatiques, cycliques conjugués ou non, aromatiques ou non. Ils peuvent notamment être choisis parmi le méthylènediphényldiisocyanate, le méthylènecyclohexanediisocyanate, l'isophorone diisocyanate, le toluène diisocyanate, le naphtalène diisocyanate, le 1,4-butanediisocyanate et le 1,6-hexane-diisocyanate, et leur mélange ; de préférence l'isophorone diisocyanate.

Préférentiellement, le ou les polyuréthanes utilisables dans la composition selon l'invention sont constitués essentiellement :
- d'au moins un motif cationique résultant d'amines de formule : 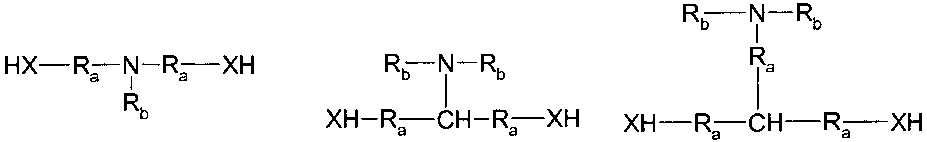 dans lesquelles :
   Rₐ est un groupe divalent alkylène en C₁-C₆, linéaire ou ramifié, notamment un groupe méthylène ou éthylène ;
   R_{b} est un groupe alkyle linéaire ou ramifié, en C₁-C₆, notamment un groupe méthyle, éthyle, n-butyle, isobutyle ou tert-butyle ;
   et X représente un atome d'hydrogène.
- d'au moins un motif non ionique résultant de polyoléfines choisies parmi les homopolymères 1,4-polybutadiène et 1,2-polybutadiène ; ou les copolymères de structure : 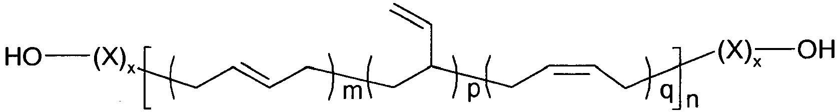 dans laquelle :
   m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
   n est un entier allant de 10 à 100, notamment de 15 à 50 ;
   x=0 ou 1, et
   X représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.
- d'au moins un motif résultant de diisocyanates aliphatiques.

Encore plus préférentiellement, le ou les polyuréthanes utilisables selon l'invention sont constitués essentiellement :
- d'au moins un motif cationique résultant d'amines de formule : 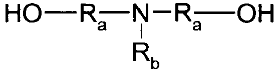 dans laquelle Rₐ est un groupe divalent alkylène en C₁-C₆, linéaire ou ramifié, notamment un groupe méthylène ou éthylène ; et R_{b} est un groupe alkyle linéaire ou ramifié, en C₁-C₆, notamment un groupe méthyle, éthyle, n-butyle, isobutyle ou tert-butyle ;
   et plus particulièrement d'au moins un motif cationique choisi parmi la N-méthyldiéthanolamine et la N-tert-butyldiéthanolamine ;
- d'au moins un motif non ionique résultant de polyoléfines de structure : 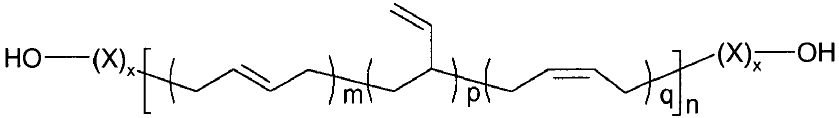 dans laquelle :
   m, p et q sont des fractions molaires allant de 0 à 1, et m+p+q=1 ; avec notamment m allant de 0,1 à 0,8, voire de 0,15 à 0,7 ; p allant de 0,1 à 0,8, voire de 0,15 à 0,7 ; et q allant de 0,05 à 0,5, voire de 0,1 à 0,4 ;
   n est un entier allant de 10 à 100, notamment de 15 à 50 ;
   x=0 ou 1, et
   X = représente un groupe carboné divalent, notamment un groupe alkylène, linéaire, cyclique ou ramifié, comprenant de 1 à 10 atomes de carbone ; tel que par exemple un groupe méthylène, éthylène, propylène ou isopropylène.
- d'au moins un motif résultant de diisocyanates choisis parmi le méthylènecyclohexanediisocyanate, l'isophorone diisocyanate, le 1,4-butanediisocyanate et le 1,6-hexane-diisocyanate ; de préférence l'isophorone diisocyanate.

Les polyuréthanes utilisables selon l'invention sont constitués essentiellement de motifs (a1), (a2) et (b) tels que définis ci-dessus, ce qui implique qu'il ne comprend pas de motifs additionnels autres que ceux-ci.

Parmi tous les polyuréthanes cités ci-dessus, on utilise de préférence, les polyuréthanes formés par les monomères suivants :
(a1) au moins un N-méthyl diéthanolamine (noté NMDEA),
(a2) au moins un copolymère d'éthylène/butylène non ionique tel que commercialisé sous la dénomination Krasol LBH-P 2000, et
(b) au moins un isophrone diisocyanate (noté IPDI).

De préférence, les amines formant les motifs cationiques (a1) représentent de 0,1 % à 50 %, en particulier de 1 % à 30 %, et mieux encore de 5 % à 20 % en poids, du poids total du polyuréthane final.

De préférence, les polyoléfines formant les motifs non ioniques (a2) représentent de 30 % à 99 % en poids, en particulier de 50 % à 90 %, et mieux encore de 60 % à 80 % en poids, du poids total de polyuréthane final.

De préférence, les composés comprenant au moins deux fonctions isocyanate, formant les motifs (b) sont présents en une quantité essentiellement stoechiométrique par rapport à la somme des amines tertiaires/quaternaires formant les motifs (a1) et des polyoléfines formant les motifs (a2).

De préférence, les composés comprenant au moins deux fonctions isocyanate formant les motifs (b) représentent de 1 % à 60 % en poids, en particulier de 5 % à 50 % en poids, et mieux encore de 15 % à 35 % en poids, du poids total du polyuréthane final.

De manière plus préférée, les polyuréthanes selon l'invention sont formés à partir de :
- 20 % à 55 %, notamment de 25 % à 50%, voire de 30 % à 47 % molaire d'amine tertiaire ou quaternaire susceptible de former les motifs (a1) ;
- 1 % à 30 %, notamment de 2 % à 25 %, voire de 3 % à 20 % molaire de polyoléfine susceptible de former les motifs (a2) ; et
- 30 % à 65 %, notamment 35 % à 60 %, voire de 45 % à 55 % molaire de composé comportant au moins deux fonctions isocyanate susceptible de former les motifs (b).

Préférentiellement, le rapport molaire entre (b) et (a1)+(a2) est proche de 1.

Ces polyuréthanes et leurs synthèses sont décrits par exemple dans la demande de brevet FR-A-289 8 603.
(14) D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, et les dérivés de la chitine.

Parmi tous les polymères cationiques susceptibles d'être utilisés dans le cadre de la présente invention, on préfère mettre en oeuvre, seuls ou en mélanges, les polymères cationiques choisis parmi les homopolymères cycliques d'alkyldiallylamine ou de dialkyldiallylammonium de la famille (7), les homopolymères et copolymères cationiques de vinylamine de la famille (12) et les polyuréthanes de la famille (13) ; et plus préférentiellement le poly(vinylamine/N-vinylformamide).

Le ou les polymères (E) sont présents dans des quantités allant de préférence de 0,01 % à 10 % en poids, en particulier de 0,05 % à 8 % en poids, et mieux encore de 0,1 % à 5 % en poids, par rapport au poids total de la composition.

### Sel(s) de zinc (F)

On entend par "sel de zinc", tout composé minéral ou organique comportant dans sa structure au moins un atome de zinc.

Le ou les sels de zinc (F) utilisés selon l'invention sont choisis de préférence parmi les sels hydrosolubles de zinc.

Au sens de la présente invention, on entend par "sel hydrosoluble de zinc", tout sel présentant une solubilité dans l'eau supérieure ou égale à 0,5 % en poids à une température de 25 °C.

Parmi les sels hydrosolubles de zinc utilisables selon la présente invention, on peut citer le sulfate de zinc, le chlorure de zinc, le lactate de zinc, le gluconate de zinc, le phénolsulfonate de zinc, le salicylate de zinc , leurs dérivés et leurs mélanges.

Le salicylate de zinc et ses dérivés selon l'invention répondent à la structure (XIV) suivante : 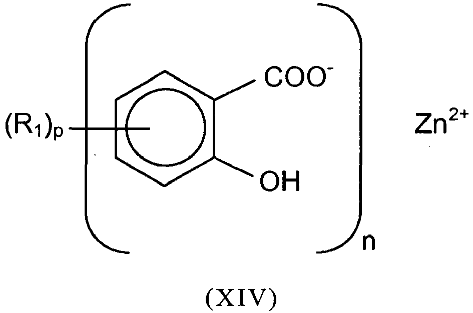 dans laquelle :
n = 2, p vaut 0, 1, 2 ou 3 ;
R₁ désigne un groupe alkyle en C₁-C₁₈ linéaire ou ramifié (par exemple méthyle, éthyle, n-propyle, isopropyle, n-butyle) ; un groupe hydroxyalkyle en C₁-C₁₈, linéaire ou ramifié ; un atome d'halogène (par exemple Iode, Brome, Chlore) ; un groupe acyle en C₂-C₁₈ (par exemple acétyle) ; un groupe COR₂, OCOR₂ ou CONHR₂ où R₂ désigne un atome d'hydrogène ou un groupe alkyle en C₁-C₁₈ linéaire ou ramifié.

On utilisera plus particulièrement le sulfate de zinc, le chlorure de zinc et/ou le gluconate de zinc vendu par exemple sous la dénomination GIVOBIO G Zn par la société SEPPIC.

De préférence, les sels de zinc de l'invention sont des sels minéraux.

On entend par "sel minéral de zinc", tout sel de zinc ne contenant éventuellement du carbone que sous forme d'ions carbonate ou hydrogénocarbonate.

Le ou les sels de zinc conformes à l'invention peuvent être présents dans la composition dans des concentrations allant de préférence de 0,005 % à 30 % en poids, en particulier de 0,1 % à 20 % en poids, et mieux encore de 0,3 % à 15 % en poids, par rapport au poids totale de la composition.

La concentration en élément zinc est de préférence inférieure à 2 % en poids, en particulier allant de 0,005 % à 1,5 % en poids, et mieux encore de 0,1 % à 1 % en poids, par rapport au poids total de la composition.

Avantageusement, le ou les sels de zinc conformes à l'invention peuvent être présents dans la composition selon l'invention dans des quantités telles que le rapport pondéral de la quantité de tensioactifs anionique(s), amphotérique(s) et/ou zwittérionique(s), et non ionique(s) sur la quantité d'élément zinc dudit ou desdits sels de zinc, est de préférence supérieure à 2, et en particulier supérieure ou égale à 5. Mieux encore, ce rapport varie de 10 à 25.

### Filtre(s) Ultra-Violet (G)

Lorsque la composition selon l'invention comprend un ou plusieurs filtres UV (systèmes filtrant les radiations Ultra-Violet), ceux-ci sont choisis parmi les filtres UV organiques ou minéraux, et sont de préférence organiques.

Le ou les filtres UV organiques utilisables selon l'invention, peuvent être choisis parmi les filtres hydrosolubles ou liposolubles, siliconés ou non siliconés.

Par "filtre hydrosoluble", on entend un filtre UV soluble dans l'eau, en particulier soluble à 25 °C à au moins 0,5 % en poids dans l'eau.

Le ou les filtres UV organiques sont notamment choisis, seul(s) ou en mélange(s), parmi :
- les dérivés de dibenzoylméthane ;
- les anthranilates ;
- les dérivés cinnamiques ;
- les dérivés salicyliques ;
- les dérivés du camphre ;
- les dérivés de la benzophénone ;
- les dérivés de bêta,bêta-diphénylacrylate ;
- les dérivés de triazine ;
- les dérivés de benzotriazole ;
- les dérivés de benzalmalonate ;
- les dérivés de benzimidazole ;
- les imidazolines ;
- les dérivés bis-benzoazolyle tels que décrits dans les brevets EP 669 323 et US 2,463,264 ;
- les dérivés de l'acide p-aminobenzoïque (PABA) ;
- les dérivés de benzoxazole tels que décrits dans les demandes de brevet EP 0 832 642, EP1 027 883, EP1 300 137 et DE 10 162 844 ;
- les polymères filtres et silicones filtres tels que ceux décrits notamment dans la demande WO 93/04665 ;
- les dimères dérivés d'alpha-alkylstyrène tels que ceux décrits dans la demande de brevet DE 19 855 649 ;
- les 4,4-diarylbutadiènes tels que décrits dans les demandes EP 0 967 200, DE 19 746 654, DE 19 755 649, EP 1 008 586, EP 1 133 980 et EP 133 981.

Comme exemples de filtres UV organiques, on peut citer ceux désignés ci-dessous sous leur nom INCI :

### Dérivés de l'acide para-aminobenzoique :

Acide p-aminobenzoïque (PABA),
Ethyl PABA,
Ethyl Dihydroxypropyl PABA,
Ethylhexyl Diméthyl PABA vendu notamment sous le nom ESCALOL 507 par la société ISP,
Glycéryl PABA,
PEG-25 PABA vendu sous le nom UVINUL P25 par la société BASF,

### Dérivés cinnamiques :

Ethylhexyl Méthoxycinnamate vendu notamment sous le nom commercial PARSOL MCX par la société HOFFMANN LA ROCHE,
Isopropyl Méthoxy cinnamate,
Isoamyl Méthoxy cinnamate vendu sous le nom commercial NEO HELIOPAN E 1000 par la société HAARMANN et REIMER,
Cinoxate,
Diéthanolamine Méthoxycinnamate,
Diisopropyl Méthylcinnamate,
Glycéryl Ethylhexanoate Diméthoxycinnamate,

### Dérivés du dibenzoylméthane :

Butyl Méthoxydibenzoylméthane vendu notamment sous le nom commercial PARSOL 1789 par la société HOFFMANN LA ROCHE,
Isopropyl Dibenzoylméthane vendu notamment sous le nom commercial EUSOLEX 8020 par la société MERCK,

### Dérivés salicyliques :

Homosalate vendu sous le nom Eusolex HMS par la société RONA/EM INDUSTRIES,
Ethylhexyl Salicylate vendu sous le nom NEO HELIOPAN OS par la société HAARMANN ET REIMER,
Dipropylèneglycol Salicylate vendu sous le nom DIPSAL par la société SCHER,
Triéthanolamine Salicylate, vendu sous le nom NEO HELIOPAN TS par la société HAARMANN ET REIMER,

### Dérivés de bêta,bêta-diphénylacrylate :

Octocrylène vendu notamment sous le nom commercial UVINUL N539 T par la société BASF,
Etocrylène, vendu notamment sous le nom commercial UVINUL N35 par la société BASF,

### Dérivés de la benzophénone :

Benzophénone-1 vendu sous le nom commercial UVINUL 400 par la société BASF,
Benzophénone-2 vendu sous le nom commercial UVINUL D50 par la société BASF,
Benzophénone-4 vendu sous le nom commercial UVINUL MS40 par la société BASF,
Benzophénone-5,
Benzophénone-6 vendu sous le nom commercial HELISORB 11 par la société NORQUAY,
Benzophénone-8 vendu sous le nom commercial SPECTRA-SORB UV-24 par la société AMERICAN CYANAMID,
Benzophénone-9 vendu sous le nom commercial UVINUL DS-49 par la société BASF,
Benzophénone-12,
2-(4-diéthylamino-2-hydroxybenzoyl)-benzoate de n-hexyle,

### Dérivés du benzylidène camphre :

3-Benzylidène camphre fabriqué sous le nom MEXORYL SD par la société CHIMEX,
4-Méthylbenzylidène camphre vendu sous le nom EUSOLEX 6300 par la société MERCK,
Acide Benzylidène Camphre Sulfonique fabriqué sous le nom MEXORYL SL par la société CHIMEX,
Méthosulfate Benzalkonium de Camphre fabriqué sous le nom MEXORYL SO par la société CHIMEX,
Acide Téréphthalylidène Dicamphre Sulfonique fabriqué sous le nom MEXORYL SX par la société CHIMEX,
Polyacrylamidométhyl Benzylidène Camphre fabriqué sous le nom MEXORYL SW par la société CHIMEX,

### Dérivés du phényl benzimidazole :

Acide Phénylbenzimidazole Sulfonique vendu notamment sous le nom commercial EUSOLEX 232 par la société MERCK,
Disodium de Phényl Dibenzimidazole Tétra-sulfonate vendu sous le nom commercial NEO HELIOPAN AP par la société Haarmann et REIMER,

### Dérivés du phényl benzotriazole :

Drométrizole Trisiloxane vendu sous le nom SILATRIZOLE par la société RHODIA,
Méthylène bis-Benzotriazolyl Tétraméthylbutylphénol, vendu sous forme solide sous le nom commercial MIXXIM BB/100 par la société FAIRMOUNT CHEMICAL ou sous forme micronisé en dispersion aqueuse sous le nom commercial TINOSORB M par la société CIBA SPECIALTY CHEMICALS,

### Dérivés de triazine :

Bis-Ethylhexyloxyphénol Méthoxyphényl Triazine vendu sous le nom commercial TINOSORB S par la société CIBA GEIGY,
Ethylhexyl triazone vendu notamment sous le nom commercial UVINUL T150 par la société BASF,
Diéthylhexyl Butamido Triazone vendu sous le nom commercial UVASORB HEB par la société SIGMA 3V,
la 2,4,6-tris-(4'-amino benzalmalonate de diisobutyle)-s-triazine,

### Dérivés anthraniliques :

Menthyl anthranilate vendu sous le nom commercial NEO HELIOPAN MA par la société HAARMANN et REIMER,

### Dérivés d'imidazolines :

Ethylhexyl Diméthoxybenzylidène Dioxoimidazoline Propionate,

### Dérivés du benzalmalonate :

Polyorganosiloxane à fonctions benzalmalonate comme le Polysilicone-15 vendu sous la dénomination commerciale PARSOL SLX par la société HOFFMANN LA ROCHE,
Diéthylsyringylidènemalonate vendu par exemple sous la dénomination commerciale OXYNEX ST par la société MERCK,

### Dérivés de 4,4-diarylbutadiène :

le 1,1-dicarboxy (2,2'-diméthyl-propyl)-4,4-diphénylbutadiène,

### Dérivés de benzoxazole :

le 2,4-bis-[5-1(diméthylpropyl)benzoxazol-2-yl-(4-phényl)-imino]-6-(2-éthylhexyl)-imino-1,3,5-triazine vendu sous le nom commercial UVASORB K2A par la société SIGMA 3V.

Avantageusement, comme filtre(s) UV organique(s) liposoluble(s) (ou lipophile(s)) convenant à une mise en oeuvre de la présente invention, on peut citer plus particulièrement :
- les dérivés de la benzophénone,
- les dérivés de bêta,bêta-diphénylacrylate,
- les dérivés du benzalmalonate,
- les dérivés de triazine,
- les dérivés du phényl benzotriazole,
- les dérivés du dibenzoylméthane,
- les dérivés du benzilidène camphre,
- et leurs mélanges.

Le ou les filtres (G) sont présents dans des quantités allant de préférence de 0,01 % à 20 % en poids, en particulier de 0,5 % à 15 % en poids, et mieux encore de 1 % à 10 % en poids, par rapport au poids total de la composition.

Dans un mode de réalisation particulier, la composition de l'invention ne comprend pas de filtre UV.

La composition selon l'invention présente de préférence un pH allant de 3 à 8. De préférence, ce pH va de 4 à 7,5. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la 1,3-propanediamine, ou encore par ajout d'un acide minéral ou organique, de préférence l'acide citrique ou l'acide chlorhydrique.

La composition selon l'invention comprend de préférence un milieu cosmétiquement acceptable.

Le milieu cosmétiquement acceptable peut être un milieu aqueux constitué uniquement par de l'eau ou par un mélange d'eau et d'un ou plusieurs solvants cosmétiquement acceptables, tel qu'un alcool inférieur en C₁-C₄, comme l'éthanol, l'isopropanol, le tertiobutanol, le n-butanol ; les alkylèneglycols comme le propylèneglycol, l'hexylèneglycol ; le glycérol ; ou leurs mélanges.

La composition selon l'invention comprend de préférence au moins 30 % en poids d'eau, en particulier de 50 % à 90 % en poids et mieux encore de 70 % à 85 % en poids, par rapport au poids total de la composition.

La composition selon l'invention peut comprendre en plus des composés (A), (B), (C), (D), (E) et (F) tels que définis précédemment, un ou plusieurs additifs.

Par "additif", on entend un composé différent des composés (A), (B), (C), (D), (E) et (F) utilisés selon l'invention et ajouté dans une composition de l'invention. En particulier, lorsque la composition selon l'invention comprend un ou plusieurs filtres UV (G) et comprend en outre un ou plusieurs additifs, ledit ou lesdits additifs étant différent(s) dudit ou desdits filtres UV (G).

Parmi les additifs utilisables, on peut citer les adjuvants classiques bien connus dans la technique, tels que les tensioactifs non ioniques autres que ceux de l'invention, les tensioactifs cationiques, les agents anti-pelliculaires, les agents anti-chute, les céramides et pseudo-céramides, les vitamines et pro-vitamines dont le panthénol, les huiles végétales, animales, minérales ou synthétiques, les cires, les pigments minéraux ou organiques, colorés ou non colorés, les colorants, les agents nacrants, les agents opacifiants, les agents séquestrants, les agents plastifiants, les agents solubilisants, les agents acidifiants, les agents alcalinisants, les agents épaississants minéraux ou organiques, les agents stabilisants, les agents antioxydants, les agents anti-radicaux libres, les parfums, les agents conservateurs, ou leurs mélanges.

En particulier, on peut utiliser parmi les agents stabilisants le tris(tétraméthylhydroxypipéridinol)citrate commercialisé sous le nom commercial TINOGARD Q par la société CIBA.

Ce ou ces additifs sont généralement chacun présents dans la composition selon l'invention en une quantité allant de 0 % à 20 % en poids, par rapport au poids total de la composition.

L'homme du métier veillera à choisir le ou les éventuels additifs et leurs quantités de manière à ce qu'ils ne nuisent pas aux propriétés des compositions de la présente invention.

Un autre objet de l'invention est un procédé de traitement cosmétique, et notamment de lavage et/ou nettoyage des fibres kératiniques, en particulier des fibres kératiniques humaines telles que les cheveux, de préférence colorées, qui comprend l'application sur lesdites fibres kératiniques d'une quantité efficace d'une composition selon l'invention.

La composition peut être appliquée sur cheveux secs ou mouillés, et de préférence sur cheveux mouillés ou humides.

Selon un mode de mise en oeuvre préféré, un tel procédé consiste à appliquer sur les cheveux une quantité efficace de la composition selon l'invention, malaxer éventuellement les cheveux, laisser pauser éventuellement la composition sur les cheveux, et rincer.

Lorsqu'on laisse pauser ladite composition sur les cheveux le temps de pause varie généralement de 0,5 à 5 minutes. La composition est généralement rincée à l'eau.

L'invention a aussi pour objet un kit de coloration comprenant un premier compartiment contenant un produit capillaire de coloration d'oxydation ou de coloration directe en une ou deux parties, et un second compartiment contenant une composition selon l'invention.

Les exemples suivants sont donnés à titre illustratif de la présente invention, et ne sauraient en limiter la portée.

### EXEMPLES

### Etape de coloration :

On prépare les mèches colorées suivantes.

Au moment de l'emploi, la coloration commerciale Majirel^{®} 6.1 (blond foncé cendré) est mélangée avec de l'eau oxygénée (à 20 volumes) poids pour poids.

Le mélange est ensuite appliqué sur des mèches de cheveux à 90 % de cheveux blancs permanentées à raison de 15 grammes de mélange colorant par gramme de mèches de cheveux. Après un temps de pause de 15 minutes, la coloration est ensuite stoppée par un rinçage à l'eau puis les mèches sont lavées à l'aide d'un shampooing commercial DOP et sont séchées 30 minutes au casque à 60°C.

### Etape de shampooings :

On a réalisé les compositions de shampooings A à G suivantes. Les quantités sont indiquées en pour cent en poids de matière active (MA) par rapport au poids total de la composition.

| **Shampooings** | **A** | **B** | **C** | **D** | **E** | **F** | **G** |
|---|---|---|---|---|---|---|---|
| Coco glucoside [1] | 5 | 5 | 5 | 5 | 5 | 5 | 5 |
| Coco bétaïne [2] | 5,4 | 5,4 | 5,4 | 5,4 | 5,4 | 5,4 | 5,4 |
| Lauryl éther carboxylate de sodium (5 OE) [3] | 3 | 3 | 3 | 3 | 3 | 3 | 3 |
| Lauryl éther sulfate (2,2 OE) de sodium [4] | 4 | 4 | 4 | 4 | 4 | 4 | 4 |
| Chlorure de poly diméthyl diallyl ammonium à 40 % en poids dans l'eau non stabilisé [5] | 1 | - | - | 1 | - | - | - |
| Poly(vinylamine/N-vinylformamide) [6] | - | 1 | - | - | 1 | - | - |
| Polyuréthane constitué de polyoléfine α-ω-dihydroxy (KRASOL LBHP 2000)/ NMDEA/ IPDI (67,9 %/ 18,7 %/ 23,4%) [7] | - | - | 1 | - | - | 1 | 1 |
| Benzophénone-4 | - | - | - | - | - | - | 5 |
| Gluconate de zinc [8] | 6,5 | 6,5 | 6,5 | - | - | - | 6,5 |
| Sulfate de zinc [9] | - | - | - | 4 | 4 | 4 | - |
| Conservateur | qs | qs | qs | qs | qs | qs | qs |
| Parfum | qs | qs | qs | qs | qs | qs | qs |
| Tampon | qs pH= 5 | qs pH= 5 | qs pH= 5 | qs pH= 5 | qs pH= 5 | qs pH= 5 | qs pH= 5 |
| Eau | qsp 100 | qsp 100 | qsp 100 | qsp 100 | qsp 100 | qsp 100 | qsp 100 |
| Rapport pondéral R [10] | 18,7 | 18,7 | 18,7 | 10,7 | 10,7 | 10,7 | 18,7 |

| | | | | | | | |
|---|---|---|---|---|---|---|---|
| [1] PLANTACARE 818 UP commercialisé par COGNIS [2] DEHYTON AB 30 commercialisé par COGNIS [3] AKYPO RLM 45 CA commercialisé par KAO [4] TEXAPON N 702 commercialisé par COGNIS [5] MERQUAT 100 commercialisé par NALCO [6] CATIOFAST VMP commercialisé par BASF [7] Polyuréthane tel que décrit dans la demande de brevet FR 2 898 603 ; le pourcentage de chacun des monomères est exprimé en poids [8] GIVOBIO G Zn commercialisé par SEPPIC [9] commercialisé par SEPPIC [10] R = (quantité de tensioactifs anionique(s), amphotérique(s) et/ou zwittérionique(s), et non ionique(s)) / (quantité d'élément zinc du sel de zinc) | | | | | | | |

### Résultats :

Ces compositions appliquées en tant que shampooings après une coloration d'oxydation permettent une meilleure ténacité de la couleur que les shampooings classiques contenant les mêmes filtres (aux mêmes quantités).

## Revendications

1. Composition cosmétique détergente destinée au lavage et/ou au nettoyage des fibres kératiniques, en particulier des fibres kératiniques humaines telles que les cheveux, notamment colorées, comprenant :
- un ou plusieurs tensioactifs anioniques (A) comprenant dans leur structure un ou plusieurs groupes sulfate et/ou sulfonate et/ou phosphate,
- un ou plusieurs tensioactifs anioniques carboxyliques (B) différents des tensioactifs anioniques cités en (A),
- un ou plusieurs tensioactifs amphotères et/ou zwittérioniques (C),
- un ou plusieurs tensioactifs non ioniques alkyl(poly)glycoside (D),
- un ou plusieurs polymères cationiques (E),
- un ou plusieurs sels de zinc (F),
- et éventuellement un ou plusieurs filtres UV (G),
et présentant un rapport pondéral de la quantité de tensioactifs anionique(s), amphotérique(s) et/ou zwittérionique(s), et non ionique(s) sur la quantité d'élément zinc dudit ou desdits sels de zinc inférieur à 30.

2. Composition selon la revendication 1, **caractérisée en ce que** le ou les tensioactif(s) (A) est (sont) présent(s) à raison de 1 % à 50 % en poids, et de préférence 2 % à 25 % en poids, par rapport au poids total de la composition.

3. Composition selon l'une quelconque des revendications précédentes, **caractérisée en ce que** le ou les tensioactif(s) (B) est (sont) présent(s) à raison de 0,5 % à 15 % en poids, et de préférence 1 % à 10 % en poids, par rapport au poids total de la composition.

4. Composition selon l'une quelconque des revendications précédentes, **caractérisée en ce que** le ou les tensioactif(s) (C) est (sont) présent(s) à raison de 0,1 % à 20 % en poids, et de préférence de 1 % à 15 % en poids, par rapport au poids total de la composition.

5. Composition selon l'une quelconque des revendications précédentes, **caractérisée en ce qu'**au moins un des rapports pondéraux, et de préférence tous les rapports pondéraux de la quantité de tensioactif(s) (A) sur la quantité de tensioactif(s) (B) ou de la quantité de tensioactif(s) (A) sur la quantité de tensioactif(s) (C), ou de la quantité de tensioactif(s) (A) sur la quantité de tensioactif(s) (D), ou de la quantité de tensioactif(s) (B) sur la quantité de tensioactif(s) (C) va de 0,1 à 10.

6. Composition selon l'une quelconque des revendications précédentes, **caractérisée en ce que** le rapport pondéral de la quantité de tensioactif(s) (B) sur la quantité de tensioactif(s) (C) va de 0,1 à 10, de préférence 0,2 à 5, et encore plus préférentiellement de 0,3 à 2.

7. Composition selon l'une quelconque des revendications précédentes, **caractérisée en ce que** le ou les tensioactif(s) (A) est (sont) choisi(s), seul(s) ou en mélange(s), parmi les alkylsulfates, les alkylamidosulfates, les alkyléthersulfates, les alkylamidoéthersulfates, les alkylaryléthersulfates, les alkyléthersulfosuccinates, les acyl iséthionates, les méthyl acyl taurates, et leurs sels ; en particulier les sels alcalins, les sels d'ammonium, les sels d'amines dont les sels d'aminoalcool, et/ou les sels de magnésium desdits composés ; le groupe alkyle ou acyle de tous ces différents composés comportant de préférence de 8 à 24 atomes de carbone, et le groupe aryle désignant de préférence un groupement phényle ou benzyle, lesdits tensioactifs pouvant être oxyéthylénés et/ou oxypropylénés.

8. Composition selon l'une quelconque des revendications précédentes, **caractérisée en ce que** le ou les tensioactif(s) (B) est (sont) choisi(s), seul(s) ou en mélange(s), parmi les composés suivants et leurs sels :
- les acides alkyl(C₆-C₂₄)-D-galactoside uroniques,
- les acides acyl(C₆-C₂₄) sarcosiniques,
- les acides acyl(C₆-C₂₄) lactyliques,
- les acides acyl(C₆-C₂₄) glutamiques,
- les acides alkyl(C₆-C₂₄) (poly)glycoside carboxyliques, tels que les acétates d'alkylglucoside, les citrates d'alkylglucoside et les tartrates d'alkylpolyglucoside,
- les acides alkyl(C₆-C₂₄) éther carboxyliques polyoxyalkylénés, en particulier ceux comportant de 2 à 50 groupements oxyde d'alkylène, de préférence de 2 à 50 groupements oxyde d'éthylène, et mieux encore de 2 à 15 groupements oxyde d'éthylène,
- les acides alkyl(C₆-C₂₄)aryl éther carboxyliques polyoxyalkylénés, en particulier ceux comportant de 2 à 50 groupements oxyde d'alkylène, de préférence de 2 à 50 groupements oxyde d'éthylène, et mieux encore de 2 à 15 groupements oxyde d'éthylène,
- les acides alkyl(C₆-C₂₄) amido éther carboxyliques polyoxyalkylénés et leurs sels, en particulier ceux comportant de 2 à 50 groupements oxyde d'alkylène, de préférence de 2 à 50 groupements oxyde d'éthylène, et mieux encore de 2 à 15 groupements oxyde d'éthylène.

9. Composition selon l'une quelconque des revendications précédentes, **caractérisée en ce que** le ou les tensioactif(s) (D) est (sont) choisi(s), seul(s) ou en mélange(s), parmi les composés répondant à la formule (IV) suivante :
R₁O-(R₂O)ₜ (G)ᵥ (IV)
dans laquelle :
R₁ représente un groupe alkyle saturé ou insaturé, linéaire ou ramifié, comportant environ de 8 à 24 atomes de carbone, un groupe alkylphényle dont le groupe alkyle linéaire ou ramifié comporte de 8 à 24 atomes de carbone,
R₂ représente un groupe alkylène comportant environ de 2 à 4 atomes de carbone,
G représente un motif saccharidique comportant de 5 à 6 atomes de carbone,
t désigne une valeur allant de 0 à 10, de préférence 0 à 4, et
v désigne une valeur allant de 1 à 15.

10. Composition selon l'une quelconque des revendications précédentes, **caractérisée en ce que** le ou les sels de zinc (F) est (sont) choisi(s) parmi les sels hydrosolubles de zinc, tels que le sulfate de zinc, le chlorure de zinc, le lactate de zinc, le gluconate de zinc, le phénolsulfonate de zinc, le salicylate de zinc, leurs dérivés et leurs mélanges.

11. Composition selon l'une quelconque des revendications précédentes, **caractérisée en ce que** le sel de zinc est un sulfate de zinc, un chlorure de zinc et/ou un gluconate de zinc.

12. Composition selon l'une quelconque des revendications précédentes, **caractérisée en ce que** le ou les sels de zinc (F) est (sont) présent(s) dans des concentrations en élément zinc inférieures à 2 % en poids, et de préférence allant de 0,005 % à 15 % en poids, du poids total de la composition.

13. Composition selon l'une quelconque des revendications précédentes, **caractérisée en ce que** le ou les sels de zinc (F) est (sont) présent(s) dans des quantités telles que le rapport pondéral de la quantité de tensioactifs anionique(s), amphotérique(s) et/ou zwittérionique(s), et non ionique(s) sur la quantité d'élément zinc dudit ou desdits composés (F) est supérieur à 2.

14. Utilisation d'une composition telle que définie dans l'une quelconque des revendications précédentes, pour le lavage et/ou le nettoyage des fibres kératiniques, en particulier des fibres kératiniques humaines telles que des cheveux, notamment colorées, et/ou protéger la couleur des fibres kératiniques colorées vis-à-vis des lavages répétés.

15. Kit de coloration comprenant un premier compartiment contenant un produit capillaire de coloration d'oxydation ou directe en une ou deux parties, et un second compartiment contenant une composition telle que définie dans l'une quelconque des revendications précédentes.

## Patentansprüche

1. Kosmetische Reinigungszusammensetzung zum Waschen und/oder Reinigen von Keratinfasern, insbesondere menschlichen Keratinfasern wie dem Haar, insbesondere gefärbtem Haar, die
- ein oder mehrere anionische Tenside (A) mit einer oder mehreren Sulfat- und/oder Sulfonat- und/oder Phosphatgruppen in ihrer Struktur,
- ein oder mehrere carboxylgruppenhaltige anionische Tenside (B), die von den in (A) aufgeführten Tensiden verschieden sind,
- ein oder mehrere amphotere und/oder zwitterionische Tenside (C),
- ein oder mehrere nichtionische Alkyl(poly)-glucosid-Tenside (D),
- ein oder mehrere kationische Polymere (E),
- ein oder mehrere Zinksalze (F)
- und gegebenenfalls ein oder mehrere UV-Schutzmittel (G)
umfasst und ein Gewichtsverhältnis der Menge von anionischem Tensid bzw. anionischen Tensiden, amphoterem Tensid bzw. amphoteren Tensiden und/oder zwitterionischem Tensid bzw. zwitterionischen Tensiden und nichtionischem Tensid bzw. nichtionischen Tensiden zur Menge des Zinkelements des Zinksalzes bzw. der Zinksalze weniger als 30 beträgt.

2. Zusammensetzung nach Anspruch 1, **dadurch gekennzeichnet, dass** das Tensid bzw. die Tenside (A) in einer Menge von 1 bis 50 Gew.-% und vorzugsweise 2 bis 25 Gew.-%, bezogen auf das Gesamtgewicht der Zusammensetzung, vorliegt bzw. vorliegen.

3. Zusammensetzung nach einem der vorhergehenden Ansprüche, **dadurch gekennzeichnet, dass** das Tensid bzw. die Tenside (B) in einer Menge von 0,5 bis 15 Gew.-% und vorzugsweise 1 bis 10 Gew.-%, bezogen auf das Gesamtgewicht der Zusammensetzung, vorliegt bzw. vorliegen.

4. Zusammensetzung nach einem der vorhergehenden Ansprüche, **dadurch gekennzeichnet, dass** das Tensid bzw. die Tenside (C) in einer Menge von 0,1 bis 20 Ges.-% und vorzugsweise 1 bis 15 Gew. -%, bezogen auf das Gesamtgewicht der Zusammensetzung, vorliegt bzw. vorliegen.

5. Zusammensetzung nach einem der vorhergehenden Ansprüche, **dadurch gekennzeichnet, dass** mindestens eines der Gewichtsverhältnisse und vorzugsweise alle der Gewichtsverhältnisse der Menge von Tensid bzw. Tensiden (A) zur Menge von Tensid bzw. Tensiden (B) oder der Menge von Tensid bzw. Tensiden (A) zur Menge von Tensid bzw. Tensiden (C) oder der Menge von Tensid bzw. Tensiden (A) zur Menge von Tensid bzw. Tensiden (D) oder der Menge von Tensid bzw. Tensiden (B) zur Menge von Tensid bzw. Tensiden (C) im Bereich von 0,1 bis 10 liegen.

6. Zusammensetzung nach einem der vorhergehenden Ansprüche, **dadurch gekennzeichnet, dass** das Gewichtsverhältnis der Menge von Tensid bzw. Tensiden (B) zur Menge von Tensid bzw. Tensiden (C) im Bereich von 0,1 bis 10, vorzugsweise 0,2 bis 5 und noch weiter bevorzugt 0,3 bis 2 liegt.

7. Zusammensetzung nach einem der vorhergehenden Ansprüche, **dadurch gekennzeichnet, dass** das Tensid bzw. die Tenside (A) aus Alkylsulfaten, Alkyl-amidosulfaten, Alkylethersulfaten, Alkylamidoethersulfaten, Alkylarylethersulfaten, Alkylethersulfosuccinaten, Acylisethionaten, Methylacyltauraten und Salzen davon; insbesondere Alkalisalzen, Ammoniumsalzen, Aminsalzen einschließlich Aminoalkoholsalzen und/oder Magnesiumsalzen der Verbindungen ausgewählt ist bzw. sind; wobei die Alkyl- oder Acylgruppe einer dieser verschiedenen Verbindungen vorzugsweise 8 bis 24 Kohlenstoffatome enthält und die Arylgruppe vorzugsweise für eine Phenyl- oder Benzylgruppe steht, wobei die Tenside oxyethyleniert oder oxypropyleniert sein können.

8. Zusammensetzung nach einem der vorhergehenden Ansprüche, **dadurch gekennzeichnet, dass** das Tensid bzw. die Tenside (B) alleine oder als Mischung bzw. Mischungen aus den folgenden Verbindungen und ihren Salzen ausgewählt sind:
- (C₆-C₂₄) -Alkyl-D-galactosiduronsäuren,
- (C₆-C₂₄) -Acylsarkosinsäuren,
- (C₆-C₂₄) -Acyllactylsäuren,
- (C₆-C₂₄) -Acylglutaminsäuren,
- (C₆-C₂₄) -Alkyl (poly) glycosidcarbonsäuren, wie Alkylglucosidacetaten, Alkylglucosidcitraten und Alkylpolyglucosidtartraten,
- polyoxyalkylenierten (C₆-C₂₄) -Alkylethercarbonsäuren, insbesondere denjenigen mit 2 bis 50 Alkylenoxidgruppen, vorzugsweise 2 bis 50 Ethylenoxidgruppen und noch besser 2 bis 15 Ethylenoxidgruppen,
- polyoxyalkylenierten (C₆-C₂₄) -Alkylarylethercarbonsäuren, insbesondere denjenigen mit 2 bis 50 Alkylenoxidgruppen, vorzugsweise 2 bis 50 Ethylenoxidgruppen und noch besser 2 bis 15 Ethylenoxidgruppen,
- polyoxyalkylenierten (C₆-C₂₄) -Alkylamidoethercarbonsäuren, insbesondere denjenigen mit 2 bis 50 Alkylenoxidgruppen, vorzugsweise 2 bis 50 Ethylenoxidgruppen und noch besser 2 bis 15 Ethylenoxidgruppen.

9. Zusammensetzung nach einem der vorhergehenden Ansprüche, **dadurch gekennzeichnet, dass** das Tensid bzw. die Tenside (D) alleine oder als Mischung bzw. Mischungen aus den Verbindungen, die der folgenden Formel (IV) entsprechen, ausgewählt sind:
R₁O- (R₂O)ₜ (G)ᵥ (IV)
wobei:
R₁ für eine lineare oder verzweigte, gesättigte oder ungesättigte Alkylgruppe mit ungefähr 8 bis 24 Kohlenstoffatomen, oder eine Alkylphenylgruppe, deren lineare oder verzweigte Alkylgruppe 8 bis 24 Kohlenstoffatome enthält, steht,
R₂ für eine Alkylengruppe mit ungefähr 2 bis 4 Kohlenstoffatomen steht,
G für eine Saccharideinheit mit 5 bis 6 Kohlenstoffatomen steht,
t für einen Wert im Bereich von 0 bis 10, vorzugsweise 0 bis 4, steht und
v für einen Wert im Bereich von 1 bis 15 steht.

10. Zusammensetzung nach einem der vorhergehenden Ansprüche, **dadurch gekennzeichnet, dass** das Zinksalz bzw. die Zinksalze (F) aus wasserlöslichen Zinksalzen, wie Zinksulfat, Zinkchlorid, Zinklactat, Zinkgluconat, Zinkphenolsulfonat und Zinksalicylat, Derivaten davon und Mischungen davon ausgewählt ist bzw. sind.

11. Zusammensetzung nach einem der vorhergehenden Ansprüche, **dadurch gekennzeichnet, dass** es sich bei dem Zinksalz um ein Zinksulfat, ein Zinkchlorid und/oder ein Zinkgluconat handelt.

12. Zusammensetzung nach einem der vorhergehenden Ansprüche, **dadurch gekennzeichnet, dass** das Zinksalz bzw. die Zinksalze (F) in Zinkelement-Konzentrationen von weniger als 2 Gew.-% und vorzugsweise im Bereich von 0,005 bis 15 Gew.-% des Gesamtgewichts der Zusammensetzung vorliegt bzw. vorliegen.

13. Zusammensetzung nach einem der vorhergehenden Ansprüche, **dadurch gekennzeichnet, dass** das Zinksalz bzw. die Zinksalze (F) in solchen Mengen vorliegt bzw. vorliegen, dass das Gewichtsverhältnis der Menge von anionischem Tensid bzw. anionischen Tensiden, amphoterem Tensid bzw. amphoteren Tensiden und/oder zwitterionischem Tensid bzw. zwitterionischen Tensiden und nichtionischem Tensid bzw. nichtionischen Tensiden zur Menge des Zinkelements der Verbindung bzw. der Verbindungen (F) mehr als 2 beträgt.

14. Verwendung einer Zusammensetzung gemäß einem der vorhergehenden Ansprüche zum Waschen und/oder Reinigen von Keratinfasern, insbesondere menschlichen Keratinfasern wie dem Haar, insbesondere gefärbtem Haar, und/oder zum Schützen der Farbe von gefärbten Keratinfasern gegenüber wiederholtem Waschen.

15. Färbekit, umfassend ein erstes Kompartiment, das ein Haarprodukt zum Oxidations- oder Direktfärben in einem oder zwei Teilen enthält, und ein zweites Kompartiment, das eine Zusammensetzung gemäß einem der vorhergehenden Ansprüche enthält.

## Claims

1. Detergent cosmetic composition intended for washing and/or cleansing keratin fibres, in particular human keratin fibres, such as the hair, especially coloured hair, comprising:
- one or more anionic surfactants (A) comprising, in their structure, one or more sulphate and/or sulphonate and/or phosphate groups,
- one or more carboxylic anionic surfactants (B) other than the anionic surfactants mentioned as (A),
- one or more amphoteric and/or zwitterionic surfactants (C),
- one or more alkyl(poly)glycoside non-ionic surfactants (D),
- one or more cationic polymers (E),
- one or more zinc salts (F),
- and optionally one or more UV-screening agents (G),
and having a weight ratio of the amount of anionic, amphoteric and/or zwitterionic, and non-ionic surfactant(s) to the amount of zinc element of said zinc salt(s) of less than 30.

2. Composition according to Claim 1, **characterized in that** the surfactant(s) (A) is (are) present in an amount of 1% to 50% by weight, and preferably 2% to 25% by weight, relative to the total weight of the composition.

3. Composition according to either one of the preceding claims, **characterized in that** the surfactant(s) (B) is (are) present in an amount of 0.5% to 15% by weight, and preferably 1% to 10% by weight, relative to the total weight of the composition.

4. Composition according to any one of the preceding claims, **characterized in that** the surfactant(s) (C) is (are) present in an amount of 0.1% to 20% by weight, and preferably 1% to 15% by weight, relative to the total weight of the composition.

5. Composition according to any one of the preceding claims, **characterized in that** at least one of the weight ratios, and preferably all of the weight ratios of the amount of surfactant(s) (A) to the amount of surfactant(s) (B) or of the amount of surfactant(s) (A) to the amount of surfactant(s) (C), or of the amount of surfactant(s) (A) to the amount of surfactant(s) (D), or of the amount of surfactant(s) (B) to the amount of surfactant(s) (C) ranges from 0.1 to 10.

6. Composition according to any one of the preceding claims, **characterized in that** the weight ratio of the amount of surfactant(s) (B) to the amount of surfactant(s) (C) ranges from 0.1 to 10, preferably from 0.2 to 5, and more preferably still from 0.3 to 2.

7. Composition according to any one of the preceding claims, **characterized in that** the surfactant(s) (A) is (are) chosen, alone or as mixture(s), from alkyl sulphates, alkylamido sulphates, alkyl ether sulphates, alkylamido ether sulphates, alkylaryl ether sulphates, alkyl ether sulphosuccinates, acyl isethionates, methyl acyl taurates, and salts thereof; in particular alkali metal salts, ammonium salts, amine salts including amino alcohol salts, and/or magnesium salts of said compounds; the alkyl or acyl group of all these various compounds preferably comprising from 8 to 24 carbon atoms, and the aryl group preferably denoting a phenyl or benzyl group, said surfactants possibly being oxyethylenated and/or oxypropylenated.

8. Composition according to any one of the preceding claims, **characterized in that** the surfactant(s) (B) is (are) chosen, alone or as mixture(s), from the following compounds and salts thereof:
- (C₆-C₂₄) alkyl D-galactoside uronic acids,
- (C₆-C₂₄)acyl sarcosinic acids,
- (C₆-C₂₄)acyl lactylic acids,
- (C₆-C₂₄) acyl glutamic acids,
- (C₆-C₂₄)alkyl(poly)glycoside carboxylic acids, such as alkylglucoside acetates, alkylglucoside citrates and alkylpolyglucoside tartrates,
- polyoxyalkylenated (C₆-C₂₄)alkyl ether carboxylic acids, in particular those comprising from 2 to 50 alkylene oxide groups, preferably from 2 to 50 ethylene oxide groups, and better still from 2 to 15 ethylene oxide groups,
- polyoxyalkylenated (C₆-C₂₄)alkylaryl ether carboxylic acids, in particular those comprising from 2 to 50 alkylene oxide groups, preferably from 2 to 50 ethylene oxide groups, and better still from 2 to 15 ethylene oxide groups,
- polyoxyalkylenated (C₆-C₂₄)alkylamido ether carboxylic acids and salts thereof, in particular those comprising from 2 to 50 alkylene oxide groups, preferably from 2 to 50 ethylene oxide groups, and better still from 2 to 15 ethylene oxide groups.

9. Composition according to any one of the preceding claims, **characterized in that** the surfactant(s) (D) is (are) chosen, alone or as mixture(s), from the compounds corresponding to the following formula (IV):
R₁O-(R₂O)ₜ (G)ᵥ (IV)
in which:
R₁ represents a linear or branched, saturated or unsaturated alkyl group comprising from about 8 to 24 carbon atoms, an alkylphenyl group in which the linear or branched alkyl group comprises from 8 to 24 carbon atoms,
R₂ represents an alkylene group comprising from about 2 to 4 carbon atoms,
G represents a sugar unit comprising from 5 to 6 carbon atoms,
t denotes a value ranging from 0 to 10, preferably from 0 to 4, and
v denotes a value ranging from 1 to 15.

10. Composition according to any one of the preceding claims, **characterized in that** the zinc salt or salts (F) is (are) chosen from water-soluble zinc salts, such as zinc sulphate, zinc chloride, zinc lactate, zinc gluconate, zinc phenolsulphonate and zinc salicylate, derivatives thereof and mixtures thereof.

11. Composition according to any one of the preceding claims, **characterized in that** the zinc salt is a zinc sulphate, a zinc chloride and/or a zinc gluconate.

12. Composition according to any one of the preceding claims, **characterized in that** the zinc salt or salts (F) is (are) present in zinc element concentrations of less than 2% by weight, and preferably ranging from 0.005% to 15% by weight, of the total weight of the composition.

13. Composition according to any one of the preceding claims, **characterized in that** the zinc salt or salts (F) is (are) present in amounts such that the weight ratio of the amount of anionic, amphoteric and/or zwitterionic, and non-ionic surfactant(s) to the amount of zinc element of said compound(s) (F) is greater than 2.

14. Use of a composition as defined in any one of the preceding claims, for washing and/or cleansing keratin fibres, in particular human keratin fibres such as hair, especially coloured keratin fibres, and/or for protecting the colour of coloured keratin fibres from repeated washing.

15. Dyeing kit comprising a first compartment containing a one-part or two-part oxidation dyeing or direct dyeing hair product, and a second compartment containing a composition as defined in any one of the preceding claims.
